# EUROPEAN PATENT APPLICATION

(11) **EP 2 540 695 A1**
(43) Date of publication of application: **02.01.2013**
(21) Application number: 11747521.0
(22) Date of filing: 25.02.2011
(51) Int. Cl.: C07C 43/23, C07C 41/42, C07C 41/46, C07D 317/54

(54) **METHOD FOR PRODUCING HIGH PURITY AROMATIC METHYL ALCOHOL, AND HIGH PURITY AROMATIC METHYL ALCOHOL COMPOSITION HAVING EXCELLENT STORAGE STABILITY**

(30) Priority: 01.03.2010 JP 2010043878; 25.02.2010 JP 2010039618
(71) Applicant: Ube Industries, Ltd., Ube-shi, Yamaguchi-ken 755-8633 (JP)
(72) Inventor: DOI, Takashi, Yamaguchi 755-863 (JP); YOSHIDA, Yoshihiro, Yamaguchi 755-863 (JP); DOUYAMA, Daisuke, Yamaguchi 755-863 (JP); KATSURA, Ryousuke, Yamaguchi 755-863 (JP); FUJITSU, Satoru, Yamaguchi 755-863 (JP); YASUDA, Shinji, Yamaguchi 755-863 (JP); KIMURA, Keisuke, Yamaguchi 755-863 (JP); OOMORI, Kiyoshi, Yamaguchi 755-863 (JP)
(74) Representative: HOFFMANN EITLE
(86) International application number: PCT/JP2011/054349
(87) International publication number: WO 2011/105565

(57) **Abstract**

An object of the present invention is to provide a high-purity aromatic methyl alcohol compound having reduced a bis(arylmethyl) ether compound as a side product mixed thereinto and a high-purity aromatic methyl alcohol composition having excellent preservation stability and methods for producing them.

The object of the present invention is achieved by a method for producing a high-purity aromatic methyl alcohol compound, which comprises obtaining a high-purity aromatic methyl alcohol compound in high yield from an aromatic methyl alcohol-containing crude product by subjecting the crude product to distillation in the presence of an anti-decomposition agent. Further, the object for the preservation stability is achieved by producing a high-purity aromatic methyl alcohol composition using the obtained high-purity aromatic methyl alcohol compound.

## Description

### FIELD OF THE INVENTION

The present invention relates to a method for producing an aromatic methyl alcohol having high purity and a high-purity aromatic methyl alcohol composition having excellent preservation stability.

### BACKGROUND ART

Aromatic methyl alcohols, which are to be produced by the present invention, are widely used as various chemical products, such as medical or agricultural chemicals and organic materials, or intermediates for syntheses thereof. Among the aromatic methyl alcohols, particularly, piperonyl alcohol, veratryl alcohol, and anise alcohol are known to be useful as a component of, e.g., perfumery and cosmetics or insecticides, or intermediates for syntheses thereof (see, for example, Patent Literatures 1 to 4).

As a method for producing the aromatic methyl alcohol, a hydrolysis reaction of an aromatic methyl halide under alkaline conditions is generally well-known.

However, it has been reported that when the hydrolysis reaction of an aromatic methyl halide is carried out under the above reaction conditions, a bis(arylmethyl) ether is by-produced, together with an aromatic methyl alcohol produced as a desired product (see, for example, Non-Patent Literature 1).
Especially when the aromatic methyl alcohol is used as a fine chemical product, such as a medical or agricultural chemical or an organic material, the substance mixed into the product may be required to be separated or removed from the product, so that the actual production process for the aromatic methyl alcohol becomes complicated.

As a method for avoiding the formation of the side product, for example, a method has been reported in which an aromatic methyl halide is reacted with sodium acetate to obtain an aromatic methyl ester of acetic acid as an intermediate, and then the obtained ester is hydrolyzed to obtain an aromatic methyl alcohol which is a desired product (see, for example, Patent Literature 5).

However, the method described in Patent Literature 5 apparently is not the industrially advantageous method, because the reaction step increases so as to go through an intermediate. Further, for example, in the above-mentioned Non-Patent Literature 1, the bis(arylmethyl) ether is known as a side product of the hydrolysis, but, with respect to the method for suppressing the formation of the ether compound, there are neither satisfactory researches or studies nor descriptions, and the problems about a commercially advantageous method for producing a high-purity aromatic methyl alcohol remain unsolved.

### PRIOR ART LITERATURES

### Patent Literatures

Patent Literature 1: Japanese Unexamined Patent Publication No. 2000-336086
Patent Literature 2: Japanese Unexamined Patent Publication No. Hei 10-121089
Patent Literature 3: Japanese Unexamined Patent Publication No. 2004-262771
Patent Literature 4: Japanese Patent Application prior-to-examination (kohyo) Publication No. 2002-531511
Patent Literature 5: International Publication No. 2005/042512 pamphlet

### Non-Patent Literature

Non-Patent Literature 1: Jingxi Huagong Zhongjianti (2004) 34(6) pages 24-26

### DISCLOSURE OF THE INVENTION

### Problems to be Solved by the Invention

With respect to a method for obtaining an aromatic methyl alcohol having high purity by removing a side product without increasing the steps for production process, distillation is generally known as a simple and effective method. However, the studies made by the present inventors show that, for example, in the production of an aromatic methyl alcohol substituted with an electron-donating group, such as a hydroxyl group, an amino group, or an alkoxy group, the aromatic methyl alcohol as a desired product suffers thermal decomposition during the distillation, and the resultant decomposition product is often mixed into the distillate fraction of the desired product, causing the purity of the obtained distillate (distillate fraction) or the distillation yield to be lowered (see, for example, Comparative Example 1 in the present invention).

Furthermore, the present inventors made studies and they have been found that even when an aromatic methyl alcohol having high purity is obtained by distillation, for example, the preserved aromatic methyl alcohol gave various decomposition products that affected by light, heat etc. Thus, they also have been found that the aromatic methyl alcohol has a problem of the stability in an environment for the preservation thereof (see, for example, Comparative Example 2 in the present invention).

Accordingly, an object of the present invention is to solve the above-mentioned problems and provide a simple method for obtaining a high-purity aromatic methyl alcohol, and a high-purity aromatic methyl alcohol composition from the obtained high-purity aromatic methyl alcohol and having excellent preservation stability.

### Means to Solve the Problems

Specifically, the object of the present invention is achieved by the invention described in items [1] to [21] shown below.

### [1]

A method for producing a high-purity aromatic methyl alcohol, which comprises a step of subjecting a crude product containing an aromatic methyl alcohol represented by the following general formula (1): 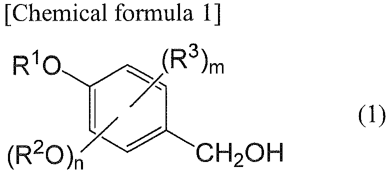 wherein
each of R¹ and R² represents a hydrogen atom, an alkyl group having 1 to 12 carbon atoms, a phenyl group, a benzyl group, an allyl group, or a propargyl group, each of which optionally has a substituent,
n is the number of substituent(s) OR² and represents an integer of from 0 to 3, and when n is 2 or more, R² may be the same or different, and when the substituents (OR¹ and OR²) on the aromatic ring are present on the adjacent carbons constituting the aromatic ring, R¹ and R² may be bonded together to form a cyclic structure,
R³ represents a halogen atom (a fluorine atom, a chlorine atom, a bromine atom, or an iodine atom), a nitro group, a cyano group, a methyl group, an ethyl group, a trifluoromethyl group, or a phenyl group, wherein when R³ and substituent OR¹ or OR² on the aromatic ring are present on the adjacent carbons constituting the aromatic ring, R³ and R¹ or R² may be bonded together to form a cyclic structure, and
m is the number of substituent(s) R³ and represents an integer of from 0 to 3, and when m is 2 or more, R³ may be the same
or different, with the proviso that n + m is an integer of from 0 to 4 to distillation in the presence of an anti-decomposition agent to obtain a high-purity aromatic methyl alcohol represented by the general formula (1).

### [2]

The method according to item [1] above, wherein the crude product containing an aromatic methyl alcohol is obtained by subjecting to hydrolysis reaction an aromatic methyl halide represented by the following general formula (2): 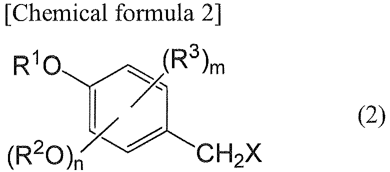 wherein R¹ to R³, n, and m are as defined for the general formula (1) in item [1] above, and X represents a chlorine atom, a bromine atom, or an iodine atom.

### [3]

The method according to item [2] above, wherein the aromatic methyl halide is selected from compounds represented by the following general formulae (2a) to (2g): 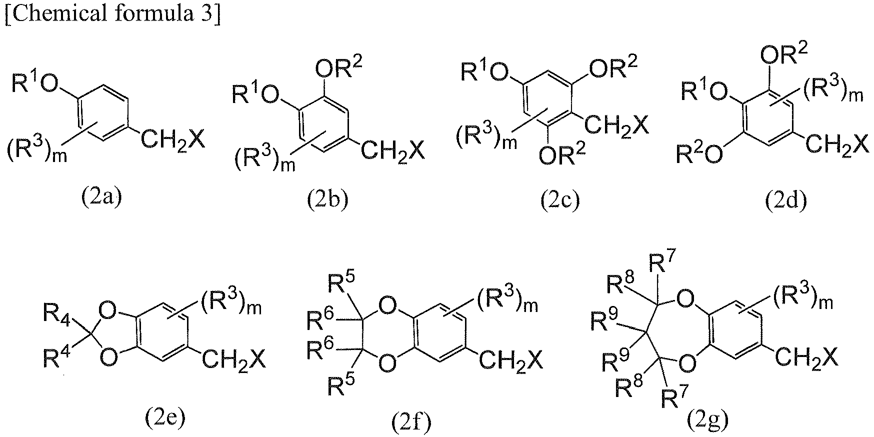 wherein R¹ to R³, X, and m are as defined for the formula (2) in item [2] above, and R¹ to R³ may be the same or different, and in the formulae (2e) to (2g), each of R⁴ to R⁹ represents a hydrogen atom, a fluorine atom, or a methyl group, and R⁴ to R⁹ may be the same or different.

### [4]

The method according to item [2] or [3] above, wherein the aromatic methyl halide is 4-methoxybenzyl chloride, 3,4-dimethoxybenzyl chloride, 3,4,5-trimethoxybenzyl chloride, 3,4-ethylenedioxybenzyl chloride, or 3,4-methylenedioxybenzyl chloride.

### [5]

The method according to any one of items [1] to [4] above, wherein the anti-decomposition agent is at least one member selected from the group consisting of an alkali metal carbonate compound, an alkaline earth metal carbonate compound, an alkali metal hydrogencarbonate compound, an alkali metal hydroxide, an alkaline earth metal hydroxide, an alkali metal phosphate compound, an alkaline earth metal phosphate compound, and an anion-exchange resin.

### [6]

The method according to any one of items [1] to [5] above, wherein the distillation is performed under conditions such that the liquid temperature in a distillation vessel is 70 to 240°C.

### [7]

The method according to any one of items [1] to [6] above, wherein the distillation is performed under conditions such that the pH of the contents of a distillation vessel is 8 to 14.
[8] A high-purity aromatic methyl alcohol being represented by the following general formula (1): 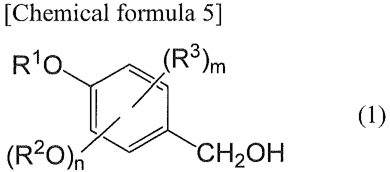 wherein R¹ to R³, n, and m are as defined for the general formula (1) in item [1] above,
in which the content of a bis(arylmethyl) ether represented by the following general formula (3): 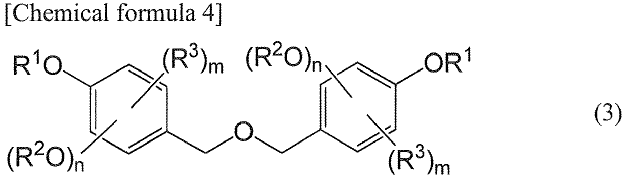 wherein R¹ to R³, n, and m are as defined for the general formula (1) in item [1] above
in the high-purity aromatic methyl alcohol is 10% or less, as determined using the following equation 1:

### [Equation 1]

$Content \left(%\right) of the bis \left(arylmethyl\right) ether represented by general formula \left(3\right) in the obtained high - purity aromatic methyl alcohol represented by general formula \left(1\right) = \frac{B}{\left(A+B\right)} \times 100 \left(%\right)$

### [9]

The high-purity aromatic methyl alcohol according to item [8] above, which is produced by the method according to any one of items [1] to [7] above.

### [10]

A high-purity aromatic methyl alcohol composition comprising a high-purity aromatic methyl alcohol represented by the following general formula (1): 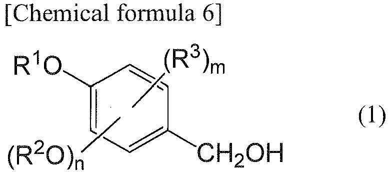 wherein R¹ to R³, n, and m are as defined for the general formula (1) in item [1] above,
and at least one anti-decomposition agent selected from the group consisting of an alkali metal carbonate compound, an alkaline earth metal carbonate compound, an alkali metal hydrogencarbonate compound, an alkali metal hydroxide, an alkaline earth metal hydroxide, an alkali metal phosphate compound, an alkaline earth metal phosphate compound, and an anion-exchange resin.

### [11]

The high-purity aromatic methyl alcohol composition according to item [10] above, wherein the high-purity aromatic methyl alcohol is the high-purity aromatic methyl alcohol according to item [9] above.

### [12]

The high-purity aromatic methyl alcohol composition according to item [10] or [11] above, wherein the amount of the anti-decomposition agent used is 200 to 50,000 ppm, based on the mass of the pure aromatic methyl alcohol contained in the high-purity aromatic methyl alcohol.

### [13]

A vessel having preserved therein the high-purity aromatic methyl alcohol composition according to any one of items [10] to [12] above.

### [14]

A method for storing a high-purity aromatic methyl alcohol, comprising a step of adding, as a preserving agent, at least one anti-decomposition agent selected from the group consisting of an alkali metal carbonate compound, an alkaline earth metal carbonate compound, an alkali metal hydrogencarbonate compound, an alkali metal hydroxide, an alkaline earth metal hydroxide, an alkali metal phosphate compound, an alkaline earth metal phosphate compound, and an anion-exchange resin to a high-purity aromatic methyl alcohol represented by the following general formula (1): 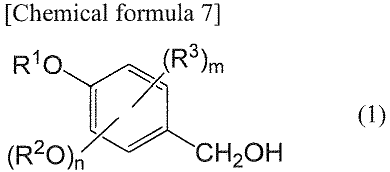 wherein R¹ to R³, n, and m are as defined for the general formula (1) in item [1] above,
to store the high-purity aromatic methyl alcohol.

### [15]

A method for distilling an aromatic methyl alcohol, comprising a step of subjecting a crude product containing an aromatic methyl alcohol represented by the following general formula (1): 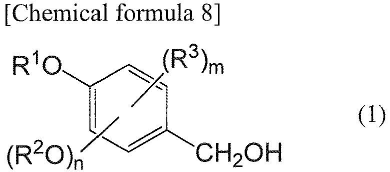 wherein R¹ to R³, n, and m are as defined for the general formula (1) in item [1] above
to distillation for purification of the aromatic methyl alcohol in the presence of at least one anti-decomposition agent selected from the group consisting of an alkali metal carbonate compound, an alkaline earth metal carbonate compound, an alkali metal hydrogencarbonate compound, an alkali metal hydroxide, an alkaline earth metal hydroxide, an alkali metal phosphate compound, an alkaline earth metal phosphate compound, and an anion-exchange resin.

### [16]

The method for distilling an aromatic methyl alcohol according to item [15] above, wherein the distillation is performed under conditions such that the pH of the contents of a distillation vessel is 8 to 14.
[17]
The method for distilling an aromatic methyl alcohol according to item [15] or [16] above, wherein the crude product containing aromatic methyl alcohol is an aromatic methyl alcohol-containing crude product obtained by subjecting to hydrolysis reaction an aromatic methyl halide represented by the following general formula (2): 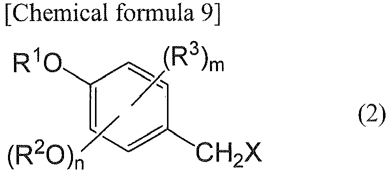 wherein R¹ to R³, n, m, and X are as defined for the general formula (2) in item [2] above.

### [18]

A method for stabilizing a high-purity aromatic methyl alcohol, comprising a step of adding, as a stabilizing agent, at least one anti-decomposition agent selected from the group consisting of an alkali metal carbonate compound, an alkaline earth metal carbonate compound, an alkali metal hydrogencarbonate compound, an alkali metal hydroxide, an alkaline earth metal hydroxide, an alkali metal phosphate compound, an alkaline earth metal phosphate compound, and an anion-exchange resin to a high-purity aromatic methyl alcohol represented by the following general formula (1): 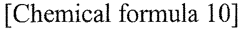 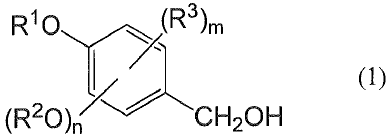 wherein R¹ to R³, n, and m are as defined for the general formula (1) in item [1] above.
[19] The method for stabilizing a high-purity aromatic methyl alcohol according to item [18] above, which comprises adding the stabilizing agent to the high-purity aromatic methyl alcohol so that the pH becomes 8 to 14.
[20]
A storage stabilizer for an aromatic methyl alcohol represented by the following general formula (1): 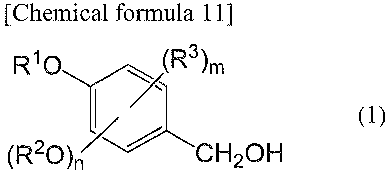 wherein R¹ to R³, n, and m are as defined for the general formula (1) in item [1] above,
the preserving agent comprising at least one member selected from the group consisting of an alkali metal carbonate compound, an alkaline earth metal carbonate compound, an alkali metal hydrogencarbonate compound, an alkali metal hydroxide, an alkaline earth metal hydroxide, an alkali metal phosphate compound, an alkaline earth metal phosphate compound, and an anion-exchange resin.

### [21]

A stabilizing agent for an aromatic methyl alcohol represented by the following general formula (1): 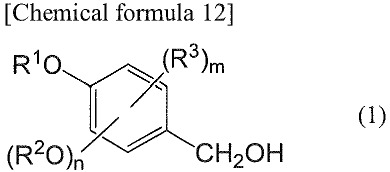 wherein R¹ to R³, n, and m are as defined for the general formula (1) in item [1] above,
comprising at least one member selected from the group consisting of an alkali metal carbonate compound, an alkaline earth metal carbonate compound, an alkali metal hydrogencarbonate compound, an alkali metal hydroxide, an alkaline earth metal hydroxide, an alkali metal phosphate compound, an alkaline earth metal phosphate compound, and an anion-exchange resin.

### Effect of the Invention

By the method for producing a high-purity aromatic methyl alcohol of the present invention, for example, the complicated production process having increased steps as described in Patent Literature 5 is not required, and the formation of various side products or decomposition products accompanying the reaction is suppressed, and thus an aromatic methyl alcohol can be produced in high yield and high purity.

Further, an aromatic methyl alcohol composition produced from the above aromatic methyl alcohol having high purity and an anti-decomposition agent can be stably preserved without causing various decomposition products due to the influence of, e.g., light or heat around the composition being preserved.

### BEST MODE FOR CARRYING OUT THE INVENTION

### «Method for producing a high-purity aromatic methyl alcohol»

The present invention is directed to a method for producing a high-purity aromatic methyl alcohol, which comprises step (A) shown in the reaction formula [I] below, and a method for producing a high-purity aromatic methyl alcohol, which comprises step (A-0) and step (A) shown in the reaction formula [II] below.

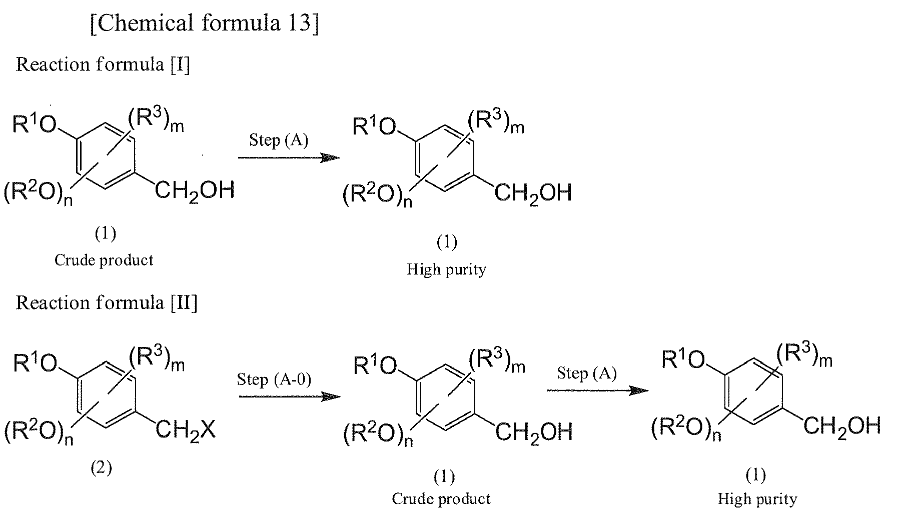

Wherein R¹ to R³, X, n, and m are as defined above.

### <Step (A)>

Step (A) in the method of the present invention is a step for the purification by distillation to obtain a high-purity aromatic methyl alcohol represented by the general formula (1) from crude product containing an aromatic methyl alcohol represented by the general formula (1), in the presence of an anti-decomposition agent.

### (Aromatic methyl alcohol-containing crude product: Starting material for step (A))

With respect to the aromatic methyl alcohol-containing crude product used as a raw material for step (A) in the present invention, there is no particular limitation as long as it contains an aromatic methyl alcohol represented by the general formula (1), but preferred is, for example, a crude product containing an aromatic methyl alcohol represented by the general formula (1) in an amount of less than 90% by mass. With respect to the aromatic methyl alcohol-containing crude product, especially preferred is any of aromatic methyl alcohol-containing crude products 1a to 1d obtained after completion of the reaction in the below-mentioned step (A-0). The crude product may contain moisture, and the moisture content of the crude product is not particularly limited. The moisture contained in the crude product is removed in the form of an azeotrope during the distillation in step (A). However, when taking into consideration the purification efficiency in the distillation in the next step, it is desired that the crude product may have some content of water without causing separation from the product.

### (Anti-decomposition agent)

The anti-decomposition agent in the present invention represents an agent used for the purpose of preventing decomposition of the aromatic methyl alcohol during the distillation in step (A) in the present invention.

As examples of the anti-decomposition agents may be used in the present invention, there can be mentioned alkaline solids, and solutions or suspensions thereof, and examples include alkali metal or alkaline earth metal carbonate compounds (e.g., lithium carbonate, sodium carbonate, potassium carbonate, calcium carbonate, and magnesium carbonate); alkali metal hydrogencarbonate compounds (e.g., lithium hydrogencarbonate, sodium hydrogencarbonate, and potassium hydrogencarbonate); alkali metal or alkaline earth metal hydroxides (e.g., lithium hydroxide, sodium hydroxide, potassium hydroxide, rubidium hydroxide, cesium hydroxide, magnesium hydroxide, calcium hydroxide, and strontium hydroxide); alkali metal or alkaline earth metal phosphate compounds (e.g., sodium phosphate, potassium phosphate, sodium hydrogenphosphate, potassium hydrogenphosphate, sodium dihydrogenphosphate, and potassium dihydrogenphosphate); and anion-exchange resins (e.g., resins having a tertiary or quaternary ammonium group, such as Amberlite IRA-400 (trade name; manufactured by Sigma-Aldrich Co. LLC.) and Amberjet 1200(trade name; manufactured by Sigma-Aldrich Co. LLC.). The anti-decomposition agents used in step (A) in the present invention may be used alone or in combination.

With respect to the anti-decomposition agent, preferably, at least one anti-decomposition agent selected from the group consisting of an alkali metal carbonate compound, an alkaline earth metal carbonate compound, an alkali metal hydrogencarbonate compound, an alkali metal hydroxide, an alkaline earth metal hydroxide, an alkali metal phosphate compound, an alkaline earth metal phosphate compound, and an anion-exchange resin is used; further preferably, an alkali metal carbonate compound, an alkali metal hydrogencarbonate compound, an alkali metal or alkaline earth metal hydroxide, or an alkali metal phosphate compound is used; further preferably, at least one anti-decomposition agent selected from the group consisting of lithium carbonate, sodium carbonate, potassium carbonate, lithium hydrogencarbonate, sodium hydrogencarbonate, potassium hydrogencarbonate, lithium hydroxide, sodium hydroxide, potassium hydroxide, rubidium hydroxide, cesium hydroxide, magnesium hydroxide, calcium hydroxide, strontium hydroxide, sodium phosphate, potassium phosphate, sodium hydrogenphosphate, potassium hydrogenphosphate, sodium dihydrogenphosphate, and potassium dihydrogenphosphate is used; more preferably, at least one anti-decomposition agent selected from the group consisting of sodium carbonate, potassium carbonate, sodium hydrogencarbonate, potassium hydrogencarbonate, lithium hydroxide, sodium hydroxide, potassium hydroxide, sodium phosphate, potassium phosphate, sodium hydrogenphosphate, and potassium hydrogenphosphate is used; and especially preferably, at least one anti-decomposition agent selected from the group consisting of sodium carbonate, potassium carbonate, lithium hydroxide, sodium hydroxide, potassium hydroxide, sodium phosphate, and potassium phosphate is used.
The anti-decomposition agent in a solid state may be used as such, and, when the aromatic methyl alcohol-containing crude product is in a liquid state or in the form of a solution, the anti-decomposition agent may be dissolved or suspended therein.

Further, it is preferred that the anti-decomposition agent in the present invention is used for achieving conditions for step (A) such that the pH of the contents of the distillation vessel in step (A) is 8 or more. Therefore, as can be seen from the above, particularly, at least one anti-decomposition agent selected from the group consisting of an alkali metal or alkaline earth metal carbonate compound, an alkali metal hydrogencarbonate compound, an alkali metal or alkaline earth metal hydroxide, and an alkali metal or alkaline earth metal phosphate compound exhibits weakly alkaline through strongly alkaline properties and is advantageously used.

### [Usage of the anti-decomposition agent]

The anti-decomposition agent used in step (A) in the present invention is used in an amount required for achieving conditions for step (A) such that the pH of the contents of the distillation vessel in step (A) is preferably 8 to 14, more preferably 9 to 14, especially preferably 10 to 13. For example, as a guide for the amount, the anti-decomposition agent is preferably used in an amount of 10 to 500,000 ppm, further preferably 200 to 50,000 ppm, more preferably 200 to 30,000 ppm, especially preferably 500 to 15,000 ppm, particularly more preferably 1,000 to 10,000 ppm, based on the mass of the pure aromatic methyl alcohol contained in the aromatic methyl alcohol-containing crude product.
By using the above-mentioned amount of the anti-decomposition agent in step (A) in the present invention, a high-purity aromatic methyl alcohol can be obtained while preventing, e.g., decomposition of the aromatic methyl alcohol per se or a side reaction (see Examples 2 and 3 of the present invention).

### (Conditions for distillation for purification)

In the distillation method used in step (A) in the present invention, the material charged into a distillation vessel before the distillation is, as mentioned above, a mixture comprising the aromatic methyl alcohol-containing crude product optionally containing moisture and an anti-decomposition agent.
The distillation in step (A) in the present invention is performed under pH conditions such that the pH of the contents of a distillation vessel is preferably 8 to 14, more preferably 9 to 14, especially preferably 10 to less than 14, during the distillation from the start through the completion of distillation. The contents of the distillation vessel are appropriately sampled and the pH is checked using, for example, a pH test paper or a pH meter. The pH is controlled by similarly checking it and appropriately adding an anti-decomposition agent.

### [Distillation apparatus]

The distillation method used in step (A) in the present invention may be any of, for example, simple distillation and rectification. Each of such distillation methods may be carried out in any of a batchwise style, a semi-continuous style, and a continuous style. The distillation is required to be carried out while finely adjusting the content of the high-purity aromatic methyl alcohol (general formula (1)) as a desired product in the main fraction, and therefore it is preferred that the apparatus for distillation is provided with a rectifying column. With respect to the rectifying column, one which is used in generally distillation, e.g., a tray type rectifying column or a packing type rectifying column can be used, and, with respect to the number of rectifying columns and the number of distillation, there is no particular limitation.

Further, in the distillation in step (A) in the present invention, with respect to a heating method for the distillation apparatus, there is no particular limitation, and, for example, a heat exchanger generally used, such as a heat exchanger of a jacket type, a coil type, a fall film type, or a thin film type, can be used. In this case, for suppressing the thermal decomposition of the aromatic methyl alcohol per se, it is preferred to use an apparatus having, as a heating apparatus, a thin film evaporator or a fall film type evaporator, in which the residence time during which the aromatic methyl alcohol is in contact with a heat transfer surface is short, connected to a rectifying column. Further, when, for example, the tray type rectifying column is used, there is no particular limitation with respect to the type of the tray.

Further, the number of the actual trays of the distillation column used in step (A) in the present invention is preferably 1 to 200, more preferably 2 to 120, especially preferably 3 to 70. In step (A) in the present invention, it has been found that when the number of the actual trays falls at least in the above-mentioned range, the separation efficiency and distillation efficiency are excellent.

### [Reflux ratio]

The reflux ratio in the distillation purification in step (A) in the present invention can be appropriately determined by checking the separation condition in each rectifying column. However, an excess reflux ratio disadvantageously requires heating for a prolonged period of time, thus promoting the decomposition of the aromatic methyl alcohol represented by the general formula (1) or other side reactions. Therefore, in the distillation in step (A) in the present invention, the reflux ratio (= reflux amount/distillate amount) is preferably 0 to 50, further preferably 0.1 to 30, especially preferably 1 to 15.

### [Packing]

When the packing type rectifying column is used, there is no particular limitation with respect to the type of the packing. However, when the distillation temperature is raised, the aromatic methyl alcohol is likely to decompose, and therefore it is preferred that a structured packing, which does not require a high liquid temperature in a distillation vessel, is used so that the pressure difference between the top and bottom of the rectifying column becomes small.

Examples of the available structured packing include "Sluzer Packing" (wire mesh type) and "Mellapack" (porous metal sheet type), each manufactured by Sluzer Chemtech Ltd.; "GEMPAK", manufactured by Koch-Glitsch, LP.; "Montz-Pak", manufactured by Montz GmbH; "Goodroll Packing", manufactured by NIPPON FILCON CO., LTD.; "Honeycomb Pack", manufactured by NGK INSULATORS, LTD.; "Impulse Packing", manufactured by Nagaoka Corporation; MC Pack (wire mesh type or metal sheet type), and Techno Pack. With respect to the materials for the rectifying column and packing, those used in general distillation, which are formed from, for example, stainless steel, Hastelloy, ceramic, or a resin, can be used.

### [Distillation temperature]

In the distillation purification in step (A) in the present invention, the liquid temperature in a distillation vessel of the distillation apparatus is appropriately selected depending on the state of the formation of a side product bis(arylmethyl) ether represented by the general formula (3). However, when the distillation temperature is raised, the aromatic methyl alcohol is likely to decompose, and therefore the distillation temperature is preferably 70 to 240°C, further preferably 90 to 210°C.

Specifically, when the aromatic methyl alcohol represented by the general formula (1) is piperonyl alcohol, bis(3,4-methylenedioxybenzyl) ether represented by the following formula (3d):

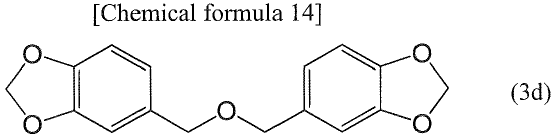

is often mixed into piperonyl alcohol. In this case, for preventing the above compound from mixing into piperonyl alcohol, the liquid temperature in the distillation vessel of the distillation apparatus is preferably 70 to 240°C, more preferably 90 to 210°C, especially preferably 100 to 180°C. Particularly, when the distillation is carried out in a batchwise style, the residence time of piperonyl alcohol in the rectifying column is longer, as compared to that in a rectification method in a continuous style, and therefore a side product of the formula (3d) is more likely to be formed due to the thermal decomposition of piperonyl alcohol, causing a problem in that the distillation yield is lowered and further the side product accompanied by a desired product is mixed into the distillation fraction. To solve the above contamination problem, when distillation purification of piperonyl alcohol is carried out in a batchwise style, the liquid temperature in the distillation vessel of the distillation apparatus is preferably 90 to 210°C, further preferably 100 to 190°C, especially preferably 110 to 180°C. The feeding of the raw material for distillation, the removing of the fraction (e.g., initial fraction) and the collecting the final product (main fraction), and the preservation of the final product, for example, can be carried out under an ordinary environment in ambient pressure, preferably under an inert gas environment, more preferably under an environment in which an inert gas is fed into the system even during the vacuum distillation, or under a stream of an inert gas.

### [Distillation pressure]

In the distillation in step (A) in the present invention, the pressure in the distillation apparatus is appropriately selected depending on the liquid temperature in a distillation vessel of the distillation apparatus and the state of the formation of a side product, such as a bis(arylmethyl) ether represented by the general formula (3).
With respect to piperonyl alcohol as a specific example of the aromatic methyl alcohol represented by the general formula (1), when the liquid temperature in the distillation vessel of the distillation apparatus is higher than 240°C, the decomposition of piperonyl alcohol per se, the formation of a side product bis(3,4-methylenedioxybenzyl) ether represented by the formula (3d), and other reactions are promoted, leading to a danger that the resultant products are disadvantageously mixed into the main fraction. Therefore, when the distillation purification of the piperonyl alcohol-containing crude product is carried out, taking into consideration the boiling point 133°C/0.66 kPa (133°C/5 Torr) of piperonyl alcohol. Thus, the distillation pressure in step (A) in the present invention is preferably 0.013 to 26.6 kPa (0.1 to 200 Torr), further preferably 0.066 to 13.3 kPa (0.5 to 100 Torr), especially preferably 0.50 to 3.9 kPa (1 to 30 Torr).

### <Aromatic methyl alcohol obtained by the present invention>

By the above-described method of the present invention, an aromatic methyl alcohol having high purity can be produced in high yield using a simple operation in an advantageous reaction time, for example, while suppressing the formation or mixing of the side product of the general formula (3).

### <Content of the bis(arylmethyl) ether (general formula (3)) in the high-purity aromatic methyl alcohol>

The high-purity aromatic methyl alcohol represented by the general formula (1), which is obtained by step (A) in the present invention, may have a bis(arylmethyl) ether represented by the general formula (3) as an impurity mixed thereinto during the distillation. Therefore, for preliminarily confirming that a high-purity aromatic methyl alcohol can be obtained by the method of the present invention, it is desired that the content of the side product represented by the general formula (3) mixed into a crude product containing an aromatic methyl alcohol represented by the general formula (1) is determined.

The content of the side product (bis(arylmethyl) ether) represented by the general formula (3) in the high-purity aromatic methyl alcohol of the present invention represented by the general formula (1) is determined using the (Equation 1) below. In (Equation 1), with respect to the detection wavelength for HPLC, a wavelength at which both a desired high-purity aromatic methyl alcohol and a side product represented by the general formula (3) can be detected is appropriately selected and used. In the present invention, in the below-mentioned Examples and Comparative Examples, a detection wavelength (λmax) of 256 nm was used in the measurement. The amount of the pure aromatic methyl alcohol and the amount of the side product represented by the general formula (3) are determined by an absolute calibration curve method.

### [Equation 2]

$Content \left(%\right) of the bis \left(arylmethyl\right) ether represented by general formula \left(3\right) in the obtained high - purity aromatic methyl alcohol represented by general formula \left(1\right) = \frac{B}{\left(A+B\right)} \times 100 \left(%\right)$

The high-purity aromatic methyl alcohol of the present invention represented by the general formula (1) is one in which the content of the side product represented by the general formula (3) in as determined above is preferably 10% by mass or less, further preferably 7% or less, more preferably 5% or less, especially preferably 3% or less, particularly more preferably 1.5% or less. When the content of the side product represented by the general formula (3) is more than 10%, the aromatic methyl alcohol can be stably stored by the preservation method or stabilization method of the present invention. However, the aromatic methyl alcohol having a side product content of more than 10% is hardly to call a high-purity aromatic methyl alcohol.

### <Purity of the high-purity aromatic methyl alcohol>

The purity of the high-purity aromatic methyl alcohol of the present invention represented by the general formula (1) is preferably 90% or more, further preferably 93% or more, more preferably 95% or more, especially preferably 97% or more, particularly more preferably 98.5% or more. The purity is a value determined from the HPLC analysis (absolute calibration curve method) as mentioned above.

### <Recovered aromatic methyl alcohol fraction>

With respect to the initial fraction, still residue, and the like obtained by the distillation other than the high-purity aromatic methyl alcohol fraction, for example, impurities are removed by a treatment, such as filtration or washing with water, and the resultant product may be used again as an aromatic methyl alcohol-containing crude product for the raw material for distillation for step (A).

### <Step (A-0)>

Step (A-0) in the method of the present invention is a step for reacting the aromatic methyl halide represented by the general formula (2) with water to obtain a crude product containing the aromatic methyl alcohol represented by the general formula (1). The aromatic methyl alcohol-containing crude product can be preferably used as an aromatic methyl alcohol-containing crude product which is a raw material for distillation in step (A).

### (Aromatic methyl halide represented by general formula (2): Starting material in step (A-0))

An aromatic methyl halide, which is a raw material for synthesis in step (A-0) in the present invention, is represented by the following general formula (2):

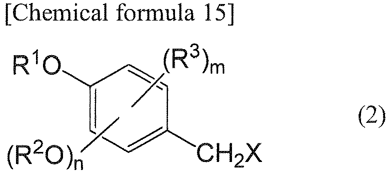

In the aromatic methyl halide represented by the general formula (2), each of R¹ and R² represents a hydrogen atom, an alkyl group having 1 to 12 carbon atoms, a phenyl group, a benzyl group, an allyl group, or a propargyl group.

The alkyl group having 1 to 12 carbon atoms for R¹ and R² represents a linear alkyl group having 1 to 12 carbon atoms (e.g., a methyl group, an ethyl group, or a n-propyl group), a branched alkyl group having 3 to 12 carbon atoms (e.g., an isopropyl group, an isobutyl group, or an isoamyl group), or a cyclic alkyl group having 3 to 12 carbon atoms (e.g., a cyclopropyl group, a cyclobutyl group, or a cyclohexyl group), and further these groups include their various isomers, such as position isomers.

Each of R¹ and R² may have a substituent(s). Examples of substituents include halogen atoms (a fluorine atom, a chlorine atom, a bromine atom, and an iodine atom), a nitro group, a cyano group, a hydroxyl group, a phenyl group, a phenyloxy group, or an alkyloxy group having 1 to 12 carbon atoms.

n is the number of substituent(s) OR² and represents an integer of from 0 to 3. When n is 2 or more, R² may be the same or different. Further, when the two substituents (for example, OR¹ and OR², or two OR²'s) on the aromatic ring are present on the adjacent carbon atoms constituting the aromatic ring, the two substituents may be bonded together to form a cyclic structure, e.g., an alkylenedioxybenzene ring, such as a methylenedioxybenzene ring or an ethylenedioxybenzene ring.

Substituent R³ represents, for example, a halogen atom (a fluorine atom, a chlorine atom, a bromine atom, or an iodine atom), a nitro group, a cyano group, a methyl group, an ethyl group, a trifluoromethyl group, or a phenyl group. When R³ and the substituent (OR¹, OR²) on the aromatic ring are present on the adjacent carbons constituting the aromatic ring, R³ and R¹ or R² may be bonded together to form a cyclic structure, e.g., a tetrahydrobenzopyran ring.
m is the number of substituent(s) R³ and represents an integer of from 0 to 3. When m is 2 or more, R³ may be the same or different, with the proviso that n + m is an integer of from 0 to 4.

In the general formula (2), X represents a chlorine atom, a bromine atom, or an iodine atom, preferably a chlorine atom or a bromine atom, further preferably a chlorine atom.

As preferred examples of the aromatic methyl halides used in the reaction in the present invention, there can be mentioned the following general formulae (2a) to (2g):

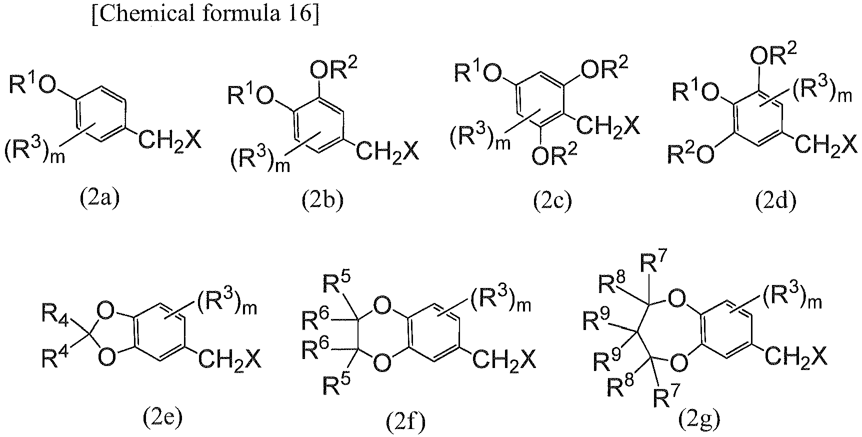

wherein R¹ to R³, X, and m are as defined above, and each of R⁴ to R⁹ represents a hydrogen atom, a fluorine atom, or a methyl group, and R⁴ to R⁹ may be the same or different.

As further preferred examples, there can be mentioned the following general formulae (2a-1), (2b-1), (2d-1), and (2e-1):

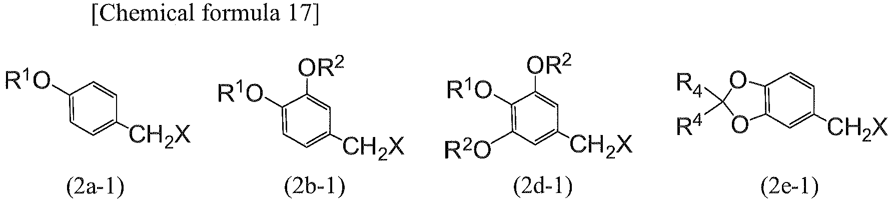

wherein R¹, R², R⁴, and X are as defined above.

More preferred examples include 4-methoxybenzyl chloride, 4-ethoxybenzyl chloride, 4-propoxybenzyl chloride, 4-cyclopropoxybenzyl chloride, 4-butoxybenzyl chloride, 4-cyclobutoxybenzyl chloride, 4-pentyloxybenzyl chloride, 4-cyclopentyloxybenzyl chloride, 4-hexyloxybenzyl chloride, 4-cyclohexyloxybenzyl chloride, 4-phenoxybenzyl chloride, 3,4-dimethoxybenzyl chloride, 3,4-diethoxybenzyl chloride, 3-methoxy-4-hydroxybenzyl chloride, 3-hydroxy-4-methoxybenzyl chloride, 3-methoxy-4-ethoxybenzyl chloride, 3-ethoxy-4-methoxybenzyl chloride, 3-benzyloxy-4-methoxybenzyl chloride, 3-methoxy-4-benzyloxybenzyl chloride, 3-allyloxy-4-methoxybenzyl chloride, 3-ethynyloxy-4-methoxybenzyl chloride, 3-methoxy-4-cyclopentyloxybenzyl chloride, 3-trifluoroethoxy-4-methoxybenzyl chloride, 3-methoxy-4-trifluoroethoxybenzyl chloride, 3,4-dihydroxybenzyl chloride, 3,4,5-trimethoxybenzyl chloride, 3,4-methylenedioxybenzyl chloride, 3,4-difluoromethylenedioxybenzyl chloride, 3,4-dimethylmethylenedioxybenzyl chloride, and 3,4-ethylenedioxybenzyl chloride.

Especially preferred examples include 4-ethoxybenzyl chloride, 4-propoxybenzyl chloride, 4-butoxybenzyl chloride, 4-cyclobutoxybenzyl chloride, 4-pentyloxybenzyl chloride, 4-cyclopentyloxybenzyl chloride, 4-hexyloxybenzyl chloride, 4-cyclohexyloxybenzyl chloride, 4-phenoxybenzyl chloride, 3,4-dimethoxybenzyl chloride, 3,4-diethoxybenzyl chloride, 3-methoxy-4-ethoxybenzyl chloride, 3-ethoxy-4-methoxybenzyl chloride, 3-benzyloxy-4-methoxybenzyl chloride, 3-methoxy-4-benzyloxybenzyl chloride, 3-allyloxy-4-methoxybenzyl chloride, 3-ethynyloxy-4-methoxybenzyl chloride, 3-methoxy-4-cyclopentyloxybenzyl chloride, 3-trifluoroethoxy-4-methoxybenzyl chloride, 3-methoxy-4-trifluoroethoxybenzyl chloride, 3,4-dihydroxybenzyl chloride, 3,4-methylenedioxybenzyl chloride, 3,4-difluoromethylenedioxybenzyl chloride, 3,4,5-trimethoxybenzyl chloride, 3,4-dimethylmethylenedioxybenzyl chloride, and 3,4-ethylenedioxybenzyl chloride.

Particularly more preferred are 4-methoxybenzyl chloride, 3,4-dimethoxybenzyl chloride, 3,4,5-trimethoxybenzyl chloride, 3,4-methylenedioxybenzyl chloride, and 3,4-ethylenedioxybenzyl chloride.

With respect to the aromatic methyl halide represented by the general formula (2), a commercially available product may be used as such, but, preferably, the aromatic methyl halide may be produced from a corresponding aromatic compound with reference to a Blanc-Quelet reaction (L. F. Fieser and M. Fieser, Advanced Organic Chemistry, p 778 (New York, 1961)).

### <Hydrolysis reaction>

### (Amount of water used)

With respect to the amount of the water used in the hydrolysis reaction in step (A-0) in the present invention, there is no particular limitation as long as the amount of the water is equimolar or more, relative to 1 mol of the above-mentioned aromatic methyl halide, however, taking into consideration the stirring efficiency during the reaction or the efficiency of the operation of isolation or purification after the reaction, usage of water is preferably in an amount of 1 to 1,000 mol, further preferably 1.25 to 500 mol, more preferably 1.5 to 250 mol, especially preferably 2.0 to 100 mol, relative to 1 mol of the aromatic methyl halide

### (Reaction solvent)

Step (A-0) in the present invention can be carried out without a solvent or in the presence of an organic solvent. The reaction system in step (A-0) may be either homogeneous or heterogeneous. Further, the homogeneous reaction system may be either of a single phase system or a multiphase system, such as a two phase system comprising aqueous-organic phases.

### [Type of the reaction solvent]

With respect to the organic solvent may be used in step (A-0) in the present invention, there is no particular limitation as long as the solvent does not prevent the reaction, and examples of the organic solvents include aliphatic hydrocarbons (e.g., n-pentane, n-hexane, n-heptane, and cyclohexane); aliphatic halides (e.g., methylene chloride and 1,2-dichloroethane); ethers (e.g., diethyl ether, diisopropyl ether, tetrahydrofuran, and dioxane); aromatic hydrocarbons (e.g., benzene, toluene, and xylene); aromatic ethers (e.g., anisole, 1,2-dimethoxybenzene, 1,3-dimethoxybenzene, 1,4-dimethoxybenzene, 1,2-methylenedioxybenzene, 1,2-ethylenedioxybenzene, and diphenyl ether); aromatic halides (e.g., chlorobenzene, 1,2-dichlorobenzene, 1,3-dichlorobenzene, and 1,4-dichlorobenzene); aromatic nitro compounds (e.g., nitrobenzene); sulfoxides (e.g., dimethyl sulfoxide); and sulfones (e.g., sulfolane). Preferably, n-pentane, n-hexane, n-heptane, cyclohexane, diethyl ether, diisopropyl ether, tetrahydrofuran, dioxane, benzene, toluene, xylene, anisole, 1,2-dimethoxybenzene, 1,3-dimethoxybenzene, 1,4-dimethoxybenzene, 1,2-methylenedioxybenzene, 1,2-ethylenedioxybenzene, diphenyl ether, chlorobenzene, dichlorobenzene, nitrobenzene, acetonitrile, propionitrile, benzonitrile, dimethyl sulfoxide, sulfolane, methylene chloride, or 1,2-dichloroethane is used. Further preferably, n-pentane, n-hexane, n-heptane, cyclohexane, diisopropyl ether, benzene, toluene, xylene, 1,2-dimethoxybenzene, 1,2-methylenedioxybenzene, chlorobenzene, or 1,2-dichloroethane is used. These solvents may be used alone or in combination.

### [Usage of the reaction solvent ]

The amount of the organic solvent used in step (A-0) in the present invention is appropriately controlled according to the uniformity or stirring efficiency of the reaction solution, but the organic solvent is used preferably in an amount of 0.1 to 1,000 mL, further preferably 0.3 to 500 mL, especially preferably 0.5 to 200 mL, relative to 1 g of the aromatic methyl halide.

### <Additive>

In step (A-0) in the present invention, an additive, such as a phase transfer catalyst or a phosphate buffer, may be additionally added to effect a reaction.

### (Phase transfer catalyst)

In step (A-0) in the present invention, for promoting the reaction, for example, the reaction may be carried out in the presence of at least one phase transfer catalyst selected from the group consisting of organic ammonium salt compounds (such as tetraethylammonium chloride, tetraethylammonium bromide, tetrabutylammonium chloride, tetrabutylammonium bromide, benzyltrimethylammonium chloride, benzyltrimethylammonium bromide, lauryltrimethylammonium chloride, and stearyltrimethylammonium chloride) and organic phosphonium salt compounds (such as tetramethylphosphonium chloride, tetrabutylphosphonium chloride, tetramethylphosphonium bromide, tetrabutylphosphonium bromide, benzyltrimethylphosphonium chloride, benzyltrimethylphosphonium bromide, tetraphenylphosphonium chloride, and tetraphenylphosphonium bromide). In the present invention, the phase transfer catalysts may be used alone or in combination. Further, the phase transfer catalyst may be used as such, or may be dissolved or suspended in, for example, water, the above-mentioned organic solvent, or a mixed solvent thereof.

### (Phosphate buffer)

In step (A-0) in the present invention, the reaction may be carried out in the presence of a phosphate buffer. Examples of phosphate buffers used in step (A-0) in the present invention include alkali metal or alkaline earth metal phosphate compounds, such as potassium phosphate, sodium phosphate, potassium monohydrogenphosphate, potassium dihydrogenphosphate, sodium monohydrogenphosphate, and sodium dihydrogenphosphate. The phosphate buffers may be used alone or in combination. Further, the phosphate buffer may be used as such, or may be dissolved or suspended in, for example, water, an organic solvent, or a mixed solvent thereof.

### (Reaction conditions: Step (A-0))

### [Reaction method]

Step (A-0) in the present invention can be performed by, for example, a method in which a mixture of the above-mentioned aromatic methyl halide and an organic solvent are mixed with water and reacted with each other while stirring, for example, in air or an inert gas atmosphere. The reaction in step (A-0) in the present invention can be carried out under any conditions of acidic conditions, neutral conditions, and alkaline conditions.

### [Reaction temperature and reaction pressure]

The reaction temperature is preferably 10 to 110°C, further preferably 0 to 100°C, especially preferably 20 to 90°C. With respect to the reaction pressure, there is no particular limitation.

### (Aromatic methyl alcohol-containing crude product in the present invention)

The aromatic methyl alcohol-containing crude product obtained in step (A-0) in the present invention can include a product in a state of any of the below-mentioned aromatic methyl alcohol-containing crude products 1a to 1d.
In the aromatic methyl alcohol-containing crude product, a bis(arylmethyl) ether which is disclosed in the above-mentioned Non-Patent Literature 1, and which is represented by the following general formula (3):

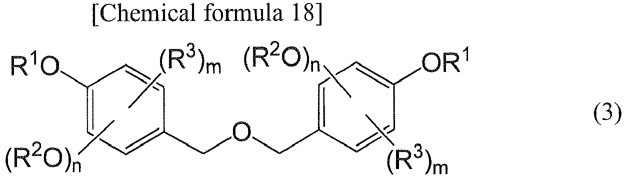

may be mixed as a side product.

### [Aromatic methyl alcohol-containing crude product 1a]

Aromatic methyl alcohol-containing crude product 1a indicates the reaction mixture obtained immediately after completion of the reaction in step (A-0) in the present invention.

### [Aromatic methyl alcohol-containing crude product 1b]

When the above-mentioned aromatic methyl alcohol-containing crude product 1a is an organic phase-aqueous phase solution comprising, for example, an aromatic methyl alcohol as a desired product and a reaction solvent, aromatic methyl alcohol-containing crude product 1b indicates an organic phase solution obtained from aromatic methyl alcohol-containing crude product 1a by an extraction and separation operation. In case of the extraction and separation operation, an available organic solvent is the same solvent as defined for the above-mentioned reaction. Further, when the crude product contains an unnecessary substance, the unnecessary substance may be removed if necessary.

More specifically, the reaction mixture (aromatic methyl alcohol-containing crude product 1a) obtained after completion of the reaction in step (A-0) in the present invention is generally in any state of a homogeneous solution, a heterogeneous solution, and a multiphase solution. For example, further operation of this reaction mixture, e.g., extraction and separation, or removal of an unnecessary substance is carried out to obtain an organic phase solution as aromatic methyl alcohol-containing crude product 1b, and the obtained crude product 1b is used as a raw material for distillation in step (A) and carried out the distillation by the method of the present invention, making it possible to reduce the operation time for the distillation.

### [Aromatic methyl alcohol-containing crude product 1c]

Aromatic methyl alcohol-containing crude product 1c indicates an organic phase solution obtained by washing the above-mentioned organic phase solution (aromatic methyl alcohol-containing crude product 1b) with an alkaline aqueous solution and so on. To remove most of the water-soluble component and acidic or alkaline component contained, the organic phase solution obtained as aromatic methyl alcohol-containing crude product 1c is appropriately washed with water, a saturated aqueous sodium chloride solution, an acidic or alkaline solution etc. And then, in case that the resultant crude product is used in the distillation in step (A), it is possible to suppress the decomposition of the aromatic methyl alcohol during the distillation. With respect to the alkaline aqueous solution used in the washing, for example, a solution obtained by dissolving the above-mentioned anti-decomposition agent in water can be used.
When the hydrolysis reaction in step (A-0) is carried out under alkaline conditions, after completion of the reaction, the resultant reaction mixture can be washed with water.

### [Aromatic methyl alcohol-containing crude product 1d]

Aromatic methyl alcohol-containing crude product 1d indicates a condensate obtained from the above-mentioned organic phase solution (aromatic methyl alcohol-containing crude product 1c), which is obtained by washing with water, a saturated aqueous sodium chloride solution, or an acidic or alkaline solution etc., by further distilling off an organic solvent, such as a reaction solvent, or a condensate obtained from aromatic methyl alcohol-containing crude product 1b (wherein the pure aromatic methyl alcohol content is 90% or more) by further distilling off a reaction solvent.
For example, the reaction solvent used in step (A-0) or the organic solvent used in the extraction and separation is removed from aromatic methyl alcohol-containing crude product 1c to obtain aromatic methyl alcohol-containing crude product 1d in the form of a condensate, and the obtained crude product 1d is used as such in the distillation purification in step (A), making it possible to reduce the operation time for the distillation purification. That is, the shorter the time for the distillation purification in step (A), the more surely the decomposition of the aromatic methyl alcohol or the mixing of a decomposition product into the distillated aromatic methyl alcohol as a desired product can be suppressed. In distilling off the reaction solvent in step (A-0), it is preferred that the above-mentioned anti-decomposition agent in step (A) is preliminarily added in a required amount to the crude product.

Further, aromatic methyl alcohol-containing crude product 1d in the present invention is purified in the treatment step for obtaining the crude product 1b from aromatic methyl alcohol-containing crude product 1a, and further, when an organic solvent or the like is distilled off from aromatic methyl alcohol-containing crude product 1d, the crude product 1d itself may exhibit a high purity (90% by mass or more) equivalent to that of the aromatic methyl alcohol of the present invention. Therefore, aromatic methyl alcohol-containing crude product 1d can be optionally used as the high-purity aromatic methyl alcohol of the present invention in the preservation by the below-mentioned method or as a raw material for the production of the high-purity aromatic methyl alcohol composition.

The relationship between aromatic methyl alcohol-containing crude products 1a to 1d is summarized as follows. Each of the obtained crude products 1a to 1d can be preferably used as a raw material for distillation in step (A) in the present invention.

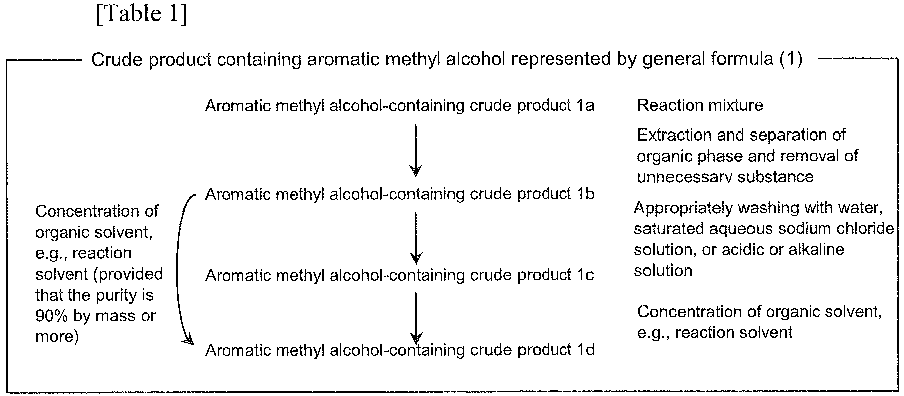

As described above, in the present invention, by the above-mentioned method comprising step (A) or method comprising step (A-0) and step (A), an aromatic methyl alcohol having high purity can be produced in high yield in an advantageous reaction time using a simple operation, for example, while suppressing the formation or mixing of the side product represented by the general formula (3).

### «High-purity aromatic methyl alcohol composition and method for producing a preservation vessel containing a high-purity aromatic methyl alcohol composition»

Next, the high-purity aromatic methyl alcohol composition of the present invention and a method for producing a preservation vessel containing a high-purity aromatic methyl alcohol composition are described. The high-purity aromatic methyl alcohol composition of the present invention is a composition comprising a high-purity aromatic methyl alcohol and an anti-decomposition agent. Regarding methods for producing the composition, method 1 and method 2 shown below may be mentioned.

### <Method 1>

Method 1 is a method in which the anti-decomposition agent in the present invention is added to a high-purity aromatic methyl alcohol in a solid state and the resultant mixture is mixed by, for example, shaking or stirring to prepare a high-purity aromatic methyl alcohol composition, and a preservation vessel is filled with the composition. The anti-decomposition agent may be added either before or after the high-purity aromatic methyl alcohol is placed in the preservation vessel.

### <Method 2>

Method 2 is a method in which when transferring a high-purity aromatic methyl alcohol melted by, for example, heating to a preservation vessel, an anti-decomposition agent is added to the melted high-purity aromatic methyl alcohol to produce a preservation vessel filled with a high-purity aromatic methyl alcohol composition, and the produced vessel is preserved. The anti-decomposition agent may be added either before or after the melted high-purity aromatic methyl alcohol is placed in the preservation vessel, but preferred is a method in which the anti-decomposition agent is added after the melted high-purity aromatic methyl alcohol is placed in the preservation vessel and before the melted high-purity aromatic methyl alcohol is solidified.

### <High-purity aromatic methyl alcohol used in the composition >

In the high-purity aromatic methyl alcohol composition of the present invention, with respect to the high-purity aromatic methyl alcohol, there can be used the high-purity aromatic methyl alcohol represented by the general formula (1), which is purified by distillation in the above-mentioned step (A), or aromatic methyl alcohol-containing crude product 1d in which the purity of the aromatic methyl alcohol represented by the general formula (1) is 95% or more.

### <Anti-decomposition agent>

With respect to the anti-decomposition agent used in the high-purity aromatic methyl alcohol composition and a production method and a preservation method therefor, an agent as defined for the agent used for the purpose of preventing decomposition of the aromatic methyl alcohol during the distillation in the above-mentioned step (A) is used. In the high-purity aromatic methyl alcohol composition and the production method and preservation method therefor, the anti-decomposition agent may be used in the form of an aqueous solution.

### (Amount of the anti-decomposition agent used)

The amount of the anti-decomposition agent used in the high-purity aromatic methyl alcohol composition of the present invention is used in an amount required for achieving conditions such that the pH of the contents of the preservation vessel is preferably 8 to 14, more preferably 9 to 14, especially preferably 10 to 13. For example, as a guide for the amount, the anti-decomposition agent is preferably used in an amount of 10 to 500,000 ppm, further preferably 200 to 50,000 ppm, more preferably 200 to 30,000 ppm, especially preferably 500 to 15,000 ppm, particularly more preferably 1,000 to 10,000 ppm, based on the amount of the pure aromatic methyl alcohol contained in the aromatic methyl alcohol-containing crude product.
The contents of the preservation vessel are appropriately sampled and the pH is checked using, for example, a pH test paper or a pH meter. The pH is controlled by checking it and appropriately adding an anti-decomposition agent as in the same style. When the usage of the anti-decomposition agent is, for example, smaller than the above range, the preventive effect for the decomposition of the aromatic methyl alcohol is not satisfactory, and, when the amount is larger than the above range, the aromatic methyl alcohol composition is in a suspension or slurry state and hence poor in handling properties, or neutralization is needed when using the aromatic methyl alcohol removed from the aromatic methyl alcohol composition, and further such a large amount is not desirable from an economical point of view.

### <Preservation vessel>

The high-purity aromatic methyl alcohol of the present invention, particularly, a high-purity aromatic methyl alcohol having an alkoxy group, such as piperonyl alcohol, veratryl alcohol, or anise alcohol, may suffer decomposition due to the influence of, for example, light or heat around it to cause decomposition products including the compound of the general formula (3) (see, for example, Comparative Example 2 in the present invention).
Therefore, it is desired that the high-purity aromatic methyl alcohol composition obtained by the above-mentioned method (method 1 or method 2) of the present invention is preserved in a light screening preservation vessel. Examples of materials for the preservation vessel include glass, ceramic, a plastic, a metal, paper, wood, bamboo, and jute, but there is no particular limitation. Alternatively, there may be used a vessel obtained from, for example, a general-purpose vessel by subjecting the inner surface of the vessel to pretreatment, such as washing with a anti-decomposition agent or a solution thereof and drying, before the use. Further alternatively, the receiver for the main fraction used in the distillation may be used as such as a preservation vessel. The inside of the preservation vessel may be filled with an inert gas, such as nitrogen, argon, or helium.

### <<Aromatic methyl alcohol composition preservation method>>

Next, the high-purity aromatic methyl alcohol preservation method is described.

### <Aromatic methyl alcohol used in the method>

When the aromatic methyl alcohol including the high-purity aromatic methyl alcohol obtained by the present invention is an aromatic methyl alcohol having an electron-donating group, particularly, an aromatic methyl alcohol having an alkoxy group, such as piperonyl alcohol, veratryl alcohol, or anise alcohol, the decomposition of the aromatic methyl alcohol being preserved can be suppressed by the below-mentioned preservation method of the present invention, irrespective of the purity of the aromatic methyl alcohol.
Therefore, the aromatic methyl alcohol used in the preservation method of the present invention is the high-purity aromatic methyl alcohol represented by the general formula (1), which is purified by distillation in the above-mentioned step (A), or aromatic methyl alcohol-containing crude product 1d in which the purity of the aromatic methyl alcohol represented by the general formula (1) is 90% or more, and the high-purity aromatic methyl alcohol composition produced by the method of the present invention. However, taking into consideration the commercial use of the aromatic methyl alcohol compound as a raw material for, e.g., a perfume, it is preferred that the preservation method is a preservation method for the high-purity aromatic methyl alcohol or high-purity aromatic methyl alcohol composition of the present invention, which is a high purity product.

### <Preservation vessel>

With respect to the material for the preservation vessel used in the preservation method of the present invention, there is no particular limitation, and examples include glass, ceramic, a plastic, a metal, paper (e.g., a paper bag or a corrugated cardboard box), wood (e.g., a wooden box), bamboo (e.g., a bamboo basket and a bamboo tube), and jute (e.g., a jute bag).

### <Preserving agent>

With respect to the preserving agent used the preservation method of the aromatic methyl alcohol of the present invention, an available preserving agent is the same that can be used for the purpose of preventing decomposition of the aromatic methyl alcohol during the distillation in the above-mentioned step (A).

### (Amount of the preserving agent used)

With respect to the usage of the preserving agent in the preservation method of aromatic methyl alcohol, there is no particular limitation within the range of pH (pH of 8 or more) described in the method for producing a high-purity aromatic methyl alcohol composition can be achieved. When the amount of the anti-decomposition agent used is too large, a complicated operation for removing the agent before fabrication is required. Therefore, for example, as a guide for the amount, the preserving agent is preferably used in an amount of 10 to 500,000 ppm, further preferably 200 to 50,000 ppm, more preferably 200 to 30,000 ppm, especially preferably 500 to 15,000 ppm, particularly more preferably 1,000 to 10,000 ppm, based on the amount of the pure aromatic methyl alcohol contained in the aromatic methyl alcohol composition. For example, when the amount of the preserving agent is smaller than the above range, the effect for preventing the decomposition of the high-purity aromatic methyl alcohol is insufficient, and, when the amount is larger than the above range, the aromatic methyl alcohol composition is in a suspension or slurry condition and hence poor in handling properties, or neutralization is needed when using the aromatic methyl alcohol removed from the aromatic methyl alcohol composition, and further such a large amount is not desirable from an economical point of view.
The high-purity aromatic methyl alcohol composition produced by the method of the present invention already contains a preserving agent in the above-mentioned amount.

### (Preservation condition)

With respect to the preservation condition of the high-purity aromatic methyl alcohol composition obtained by the present invention, there is no particular limitation, but it is desired that the inside of the preservation vessel is under a light-shielded condition and is under a stream of an inert gas, such as nitrogen, argon, or helium, or filled with a gas.

### (Preservation temperature)

With respect to the preservation of the high-purity aromatic methyl alcohol composition of the present invention, the high-purity aromatic methyl alcohol composition can be stably stored in an ordinary environment at room temperature (for example, at 0 to 50°C).
However, the high-purity aromatic methyl alcohol of the present invention, particularly, the high-purity aromatic methyl alcohol having an alkoxy group may decompose in only a heating environment even when it stored under a light shielded condition.

Therefore, it is desired that the high-purity aromatic methyl alcohol composition obtained by the present invention is stored in a solid state at a temperature of the melting point (literature value) of the high-purity aromatic methyl alcohol to be contained or lower. More specifically, taking the handling properties of the composition into consideration, the preservation temperature is preferably 100 to -10°C, more preferably 90 to -10°C, especially preferably 70 to -5°C. The high-purity aromatic methyl alcohol composition containing the high-purity aromatic methyl alcohol having a melting point of 10°C or lower may be stored either in a liquid state or in a solidified state in the environment at room temperature.

More specifically, when the high-purity aromatic methyl alcohol is piperonyl alcohol, it can be stored in the above-mentioned environment at room temperature but is preferably stored at 100 to -10°C, more preferably at 90 to -10°C, especially preferably at 70 to-10°C.

### <<Aromatic methyl alcohol stabilization method>>

The present invention relates to a high-purity aromatic methyl alcohol stabilization method, which comprises adding as a stabilizing agent the above-mentioned anti-decomposition agent, which is at least one anti-decomposition agent selected from the group consisting of an alkali metal carbonate compound, an alkaline earth metal carbonate compound, an alkali metal hydrogencarbonate compound, an alkali metal hydroxide, an alkaline earth metal hydroxide, an alkali metal phosphate compound, an alkaline earth metal phosphate compound, and an anion-exchange resin, to a high-purity aromatic methyl alcohol represented by the following general formula (1): 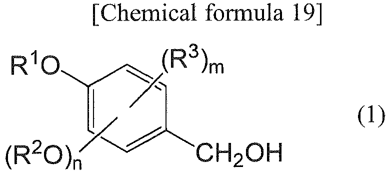 wherein R¹ to R³, n, and m are as defined for the general formula (1) in item [1] above
or the above-mentioned aromatic methyl alcohol-containing crude product. More particularly, the present invention is concerned with an aromatic methyl alcohol stabilization method, which comprises adding the stabilizing agent to the above-mentioned high-purity aromatic methyl alcohol or aromatic methyl alcohol-containing crude product so that the pH becomes 8 to 14.

### (Aromatic methyl alcohol used)

The aromatic methyl alcohol used in the present invention is the same as the <Aromatic methyl alcohol used in the method> described in «Aromatic methyl alcohol composition preservation method» above and the (Aromatic methyl alcohol-containing crude product in the present invention) described in «Method for producing an aromatic methyl alcohol» above. Taking the industrial utility into consideration, the aromatic methyl alcohol is preferably the <Aromatic methyl alcohol used in the method> and aromatic methyl alcohol-containing crude products 1b, 1c, and 1d described in (Aromatic methyl alcohol-containing crude product in the present invention).

### (Stabilizing agent)

With respect to the stabilizing agent used in the aromatic methyl alcohol stabilization method, an agent as defined for the anti-decomposition agent used in the distillation in the above-mentioned step (A) is used as a stabilizing agent for aromatic methyl alcohol.

### [Amount of the stabilizing agent used]

The amount of the stabilizing agent used is as defined for the amount of the anti-decomposition agent used in the distillation in the above-mentioned step (A). Further, like the anti-decomposition agent used in the distillation in step (A), the stabilizing agent is added to the high-purity aromatic methyl alcohol so that the pH becomes 8 to 14.

In the present invention, by using the stabilizing agent, the aromatic methyl alcohol represented by the general formula (1) can be stably present, for example, even at a temperature as high as 160°C, which corresponds to the temperature condition for the above-mentioned distillation.

### EXAMPLES

Hereinbelow, the present invention will be described in more detail with reference to the following Examples, which should not be construed as limiting the scope of the present invention. In the following Examples and Comparative Examples, "%" is given by mass unless otherwise specified.

### <Analysis conditions for high performance liquid chromatography (HPLC) in the below-shown Examples 1 to 3 and Comparative Example 1>

Column: ODS-80TsQA φ 4.6 mm x 250 mm (manufactured by TOSOH CORPORATION)
Eluent: Acetonitrile/H₂O = 270/400 [mass/mass] (pH was adjusted to 2.5 using trifluoroacetic acid)
Column temperature: 40°C
Detector: 256 nm
Flow rate: 0.6 mL/min

### <Analysis conditions for high performance liquid chromatography (HPLC) in Examples 4 to 13 and Comparative Examples 2 to 4>

Column: ODS-80TsQA φ 4.6 mm x 250 mm (manufactured by TOSOH CORPORATION)
Eluent: Acetonitrile/H₂O = 520/1,000 [mass/mass] (pH was adjusted to 2.5 using trifluoroacetic acid)
Column temperature: 40°C
Detector: 256 nm
Flow rate: 1.0 mL/min

### Reference Example 1 (Preparation of a toluene solution of piperonyl chloride)

In a glass reaction vessel equipped with a thermometer, a temperature regulator, a dropping apparatus, and a stirrer were placed 4.89 kg (40 mol) of 1,2-methylenedioxybenzene, 3.9 kg (42 mol) of toluene, 1.41 kg (43.2 mol) of paraformaldehyde, and 9.10 kg (90.8 mol) of a 36% aqueous solution of hydrochloric acid, and 9.90 kg of a toluene solution of piperonyl chloride as a desired product was obtained in accordance with the method described in Non-Patent Literature 2.

### <<Step (A-0)>>

### Example 1 (Synthesis of piperonyl alcohol crude product 1b)

In a glass reaction vessel equipped with a thermometer, a temperature regulator, a dropping apparatus, and a stirrer were placed 6.9 L of water and 32 g (0.20 mol) of sodium dihydrogenphosphate dihydrate (NaH₂PO₄•2H₂O), and the temperature of the resultant solution was allowed to become 48°C. To the solution were added 9.88 kg (20.1 mol; amount of piperonyl chloride contained: 3.45 kg) of the toluene solution of piperonyl chloride synthesized in Reference Example 1 and 2.50 kg (23.1 mol) of a 37% aqueous solution of sodium hydroxide, and the resultant mixture was stirred for 2 hours, and subsequently stirred at a temperature of 90°C for 3 hours. After completion of the reaction, the resultant reaction mixture was cooled to room temperature, and the extraction is carried out to obtain an organic phase. To the aqueous phase was added toluene, followed by extraction, to obtain an organic phase. The obtained organic phase was mixed with the previously obtained organic phase to obtain 10.4 kg of piperonyl alcohol crude product 1b in the form of a toluene solution of a piperonyl alcohol crude product as a desired product.

Values of analysis made with respect to the obtained piperonyl alcohol crude product 1b were as follows.
Amount of piperonyl alcohol contained (HPLC analysis, absolute calibration curve method): 2.73 kg.
Piperonyl alcohol content (HPLC analysis, absolute calibration curve method): 26% by mass.
Yield of piperonyl alcohol (based on the amount of the piperonyl chloride used): 88.7%.

A main side product was bis(3,4-methylenedioxybenzyl) ether represented by the following formula (3d).

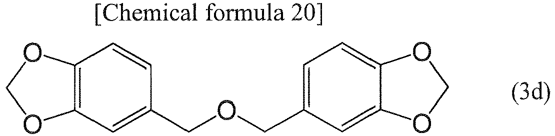

A content of the side product represented by the formula (3d) in the obtained toluene solution of piperonyl alcohol was determined by a high performance liquid chromatography analysis (absolute calibration curve method) at a detection wavelength of 256 nm from the amounts of the obtained piperonyl alcohol and the side product represented by the formula (3d) using the following (Equation 1d).

### [Equation 3]

$Content \left(%\right) of the bis ⁢ \left(3,4-methylenedioxybenzyl\right) ether represented by general formula \left(3⁢d\right) in the piperonyl alcohol crude product = \frac{{B}_{d}}{\left({A}_{d}+{B}_{d}\right)} \times 100 \left(%\right)$

As a result, the side product content was found to be 3.8%.

### Reference Example 2 (Purification of piperonyl alcohol)

Next, a portion of the toluene solution of piperonyl alcohol obtained by the same method as in Example 1 was purified by silica gel column chromatography to obtain piperonyl alcohol in the form of white solids. In the present invention, the obtained piperonyl alcohol was used as an analysis standard sample for the HPLC analysis.

The values of physical properties of the obtained compound (piperonyl alcohol) were as follows.
MS spectrum [CI-MS]: 152 [M+1]
¹H-NMR spectrum [300 MHz, CDCl₃, σ (ppm)]: 1.83 (1H, brs), 4.56 (2H, s), 5.94 (2H, s), 6.78 - 6.85 (3H, m).
Melting point: 48.0 to 53.5°C

### Example 2 (Distillation for high-purity piperonyl alcohol: step (A); anti-decomposition agent: sodium carbonate)

In a glass reaction vessel equipped with a thermometer, a temperature regulator, a dropping apparatus, and a stirrer was placed 577.32 g of the toluene solution containing 130.24 g of pure piperonyl alcohol (piperonyl alcohol crude product 1b), which had been prepared by the same method as in Example 1. Then, to the toluene solution was added 2.89 g of sodium carbonate as a anti-decomposition agent (in an amount of 22,000 ppm, based on the mass of the pure piperonyl alcohol contained in the piperonyl alcohol crude product), and the solvent was distilled off under a reduced pressure. Then, the simple distillation of the resultant residue was carried out under conditions such that the vessel temperature was 127°C and the degree of vacuum was 0.64 kPa (4.8 Torr), obtaining 125.92 g of piperonyl alcohol having a purity of 98.0% as a main fraction (recovery: 94.8%). The pH of the contents of the vessel during the distillation was about 10 as measured using a pH test paper. The total amount of the piperonyl alcohol contained in the main fraction, still residue, and solvent was 130.24 g (recovery: 100%). Further, it was found that bis(3,4-methylenedioxybenzyl) ether represented by the formula (3d) was not mixed into the obtained piperonyl alcohol.

### Example 3 (Distillation for high-purity piperonyl alcohol: step (A); anti-decomposition agent: sodium hydroxide)

Distillation was performed under substantially the same conditions as in Example 2 except that sodium hydroxide was added as an anti-decomposition agent in an amount of 0.1 wt%, based on the mass of the pure piperonyl alcohol. As a result, piperonyl alcohol having a purity of 98.9% was obtained as a main fraction at a yield of 94.3%. The pH of the contents of the distillation vessel during the distillation was about 10 as measured using a pH test paper. The total amount of the piperonyl alcohol contained in the main fraction, still residue, and solvent was 130.24 g (recovery: 100%). Further, it was found that bis(3,4-methylenedioxybenzyl) ether represented by the formula (3d) was not mixed into the obtained piperonyl alcohol.

### Comparative Example 1 (Synthesis of high-purity piperonyl alcohol; anti-decomposition agent: none)

Distillation was performed under the same conditions as in Example 2 except that no anti-decomposition agent was added. As a result, piperonyl alcohol having a purity of 96.2% was obtained as a main fraction at a yield of 82.8%. The recovery determined from the total amount of the piperonyl alcohol contained in the main fraction, still residue, and solvent was as small as 83.5%, which has confirmed that 16.5% of the piperonyl alcohol decomposes during the distillation operation in step (A). Further, it was found that bis(3,4-methylenedioxybenzyl) ether represented by the formula (3d) was mixed in an amount as large as 1.64% into the piperonyl alcohol obtained under the same conditions for obtaining the main fraction as those in Example 2.
The pH of a portion of the contents of the distillation vessel was determined by using a pH meter. As a result, it was found that the pH of the contents of the distillation vessel before the start of distillation was 6.0 and the pH of the contents of the distillation vessel after the start of distillation was 2.6.

### «High-purity aromatic methyl alcohol stabilization method»

### Example 4 (Decomposition inhibition effect of the addition of an anti-decomposition agent (sodium carbonate): piperonyl alcohol)

Toluene was distilled off from the toluene solution of piperonyl alcohol obtained by the same method as in Reference Example 1 and Example 1 to obtain a piperonyl alcohol condensate. Then, to the obtained condensate was added sodium carbonate as an anti-decomposition agent in an amount of 1,000 ppm to prepare a sample for the measurement of stabilization.
The prepared sample was placed in a thermostat at a temperature of 140°C, and a high performance liquid chromatography analysis (HPLC: absolute calibration curve method) was made with respect to the sample at intervals (at points in time of 4 hours, 8 hours, 12 hours, 16 hours, and 20 hours after the start) to measure a purity of piperonyl alcohol (in terms of % by mass). A preservation ratio of piperonyl alcohol was determined from: [HPLC purity (area %) of piperonyl alcohol after each period of time]/[HPLC purity (area %) of piperonyl alcohol at the start of measurement] × 100 (%). That is, the preservation ratio of piperonyl alcohol at the start of measurement is taken as 100%, and with respect to the value of preservation ratio after a lapse of a certain time, the higher the value, the more stably the piperonyl alcohol can be preserved. The results are shown in Table 1 below.

### Comparative Example 2 (Preservation stability of high-purity piperonyl alcohol (140°C); anti-decomposition agent: none)

As a comparative example for Example 4, an experiment the same as in Example 4, except that an anti-decomposition agent (sodium carbonate) was not used, was carried out. The results are shown in Table 2 below.

**[Table 2]**

| Thermal stability of high-purity aromatic methyl alcohol (Piperonyl alcohol) | | |
|---|---|---|
| Preservation time (Hours) | Example 4 (Anti-decomposition agent: 1,000 ppm) | Comparative Example 2 (Anti-decomposition agent: none) |
| | Piperonyl alcohol preservation ratio (%)*1 | Piperonyl alcohol preservation ratio (%)*1 |
| 4 | 99.66 | 76.24 |
| 8 | 99.49 | 57.66 |
| 12 | 100.00 | 49.19 |
| 16 | 99.10 | 43.56 |
| 20 | 99.40 | 39.48 |
| 24 | 100.00 | 36.97 |

| | | |
|---|---|---|
| *1: Preservation ratio (%) = [HPLC purity (area %) of piperonyl alcohol after each period of time]/[HPLC purity (area %) of piperonyl alcohol at the start of measurement] × 100 (%). | | |

### Examples 5 to 8 (Decomposition inhibition effect of the addition of an anti-decomposition agent (sodium carbonate): piperonyl alcohol)

A toluene solution of piperonyl alcohol having a piperonyl alcohol purity of 96.4% (HPLC: absolute calibration curve method) was obtained by the same method as in Reference Example 1 and Example 1. Then, the obtained toluene solution was placed individually in 20 ml glass flasks, and sodium carbonate as a anti-decomposition agent was added to the toluene solution in the individual flasks in respective amounts of 188 ppm (Example 5), 489 ppm (Example 6), 710 ppm (Example 7), and 950 ppm (Example 8), followed by distilling off of toluene, to prepare samples for the measurement of stabilization.
Then, each sample for the measurement of stabilization was placed in a thermostat at a temperature of 160°C, and a high performance liquid chromatography analysis (HPLC: absolute calibration curve method) was made with respect to each sample at intervals (at points in time of 7 hours, 14 hours, and 21 hours after the start) to measure a purity of piperonyl alcohol (in terms of % by mass). The results are shown in Table 3 below.

### Comparative Example 3 (Preservation stability of high-purity piperonyl alcohol (160°C); anti-decomposition agent: none)

As a comparative example for Examples 4 to 7, an experiment the same as in Examples 4 to 7, except that an anti-decomposition agent (sodium carbonate) was not used, was carried out. The results are shown in Table 3 below.

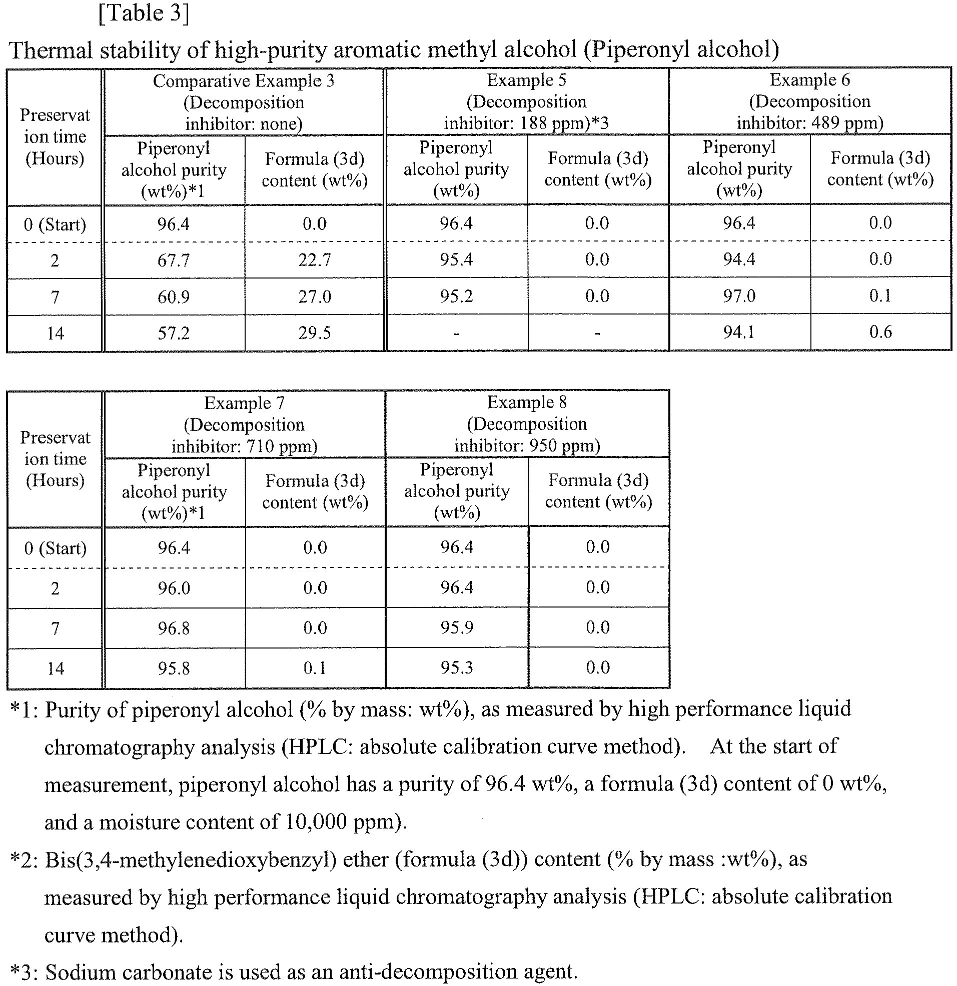

### Example 9 (Decomposition inhibition effect of the addition of a anti-decomposition agent (sodium carbonate: 10,000 ppm): p-anise alcohol)

To p-anise alcohol (manufactured by Wako Pure Chemical Industries, Ltd.) was added sodium carbonate as an anti-decomposition agent in an amount of 10,000 ppm to prepare a sample for the measurement of stabilization.
Then, the prepared sample for the measurement of stabilization was placed in a thermostat at a temperature of 160°C, and a high performance liquid chromatography analysis (HPLC: absolute calibration curve method) was made with respect to the sample at intervals (at points in time of 5 hours and 10 hours after the start) to measure a preservation ratio (%) of p-anise alcohol. The preservation ratio is determined by the same method as that in Example 4. Further, like Example 4, the preservation ratio of p-anise alcohol at the start of measurement is taken as 100%, and with respect to the value of preservation ratio after a lapse of a certain time, the higher the value, the more stably the p-anise alcohol can be stored. The results are shown in Table 4 below.

### Comparative Example 4 (Decomposition inhibition effect of the addition of an anti-decomposition agent (none): p-anise alcohol)

As a comparative example, an experiment the same as in Example 8, except that an anti-decomposition agent (sodium carbonate) was not used, was carried out. A preservation ratio is determined by the same method as that in Example 4. The results are shown in Table 4 below. The results shown in Table 4 have confirmed that about 10% of p-anise alcohol decomposes in a period of time as small as 10 hours in Comparative Example 4 using no anti-decomposition agent.

**[Table 4]**

| Stability of high-purity aromatic methyl alcohol (p-Anise alcohol) | | |
|---|---|---|
| Preservation time (Hours) | Example 9 (Anti-decomposition agent: 10,000 ppm) | Comparative Example 4 (Anti-decomposition agent: none) |
| | p-Anise alcohol preservation ratio * 1 | p-Anise alcohol preservation ratio*1 |
| 5 | 98.9 | 94.3 |
| 10 | 99.2 | 91.5 |

| | | |
|---|---|---|
| *1: Preservation ratio (%) = [HPLC purity (area %) of piperonyl alcohol after each period of time]/[HPLC purity (area %) of piperonyl alcohol at the start of measurement] × 100 (%). | | |

### «High-purity aromatic methyl alcohol preservation method»

### Example 10 (Preservative stability of high-purity piperonyl alcohol (90°C): method (2); anti-decomposition agent: sodium carbonate)

5 g of high-purity piperonyl alcohol obtained by the same method as in Example 2 was melted by heating and placed in a 30 mL glass vessel, and further 0.025 g (5,000 ppm, based on the amount of the high-purity piperonyl alcohol used) of sodium carbonate was added to the vessel. The vessel containing the resultant high-purity piperonyl alcohol composition was closed, and allowed to stand so that the composition was cooled and solidified to prepare a preservation vessel for the high-purity piperonyl alcohol composition in a solid form. Then, the vessel was carried out the test in which the vessel was preserved in a thermostat at 90°C for 28 days (the pH of the contents at the start of preservation was about 10 as measured using a pH test paper). 7 Days, 14 days, 21 days, and 28 days after the start of preservation at the above-mentioned temperature, the composition was carried out the high performance liquid chromatography analysis (HPLC: absolute calibration curve method) to determine a purity of piperonyl alcohol, in terms of a pure piperonyl alcohol content (% by mass). The results are shown in Table 5 below.

### Example 11 (Preservation stability of high-purity piperonyl alcohol): method (2); anti-decomposition agent: sodium carbonate)

Preservation was performed by the same method as in Example 10 except that the preservation temperature was changed to 45°C, and similarly, 7 days, 14 days, 21 days, and 28 days after the start of preservation, a purity of piperonyl alcohol (in terms of % by mass) was determined by a high performance liquid chromatography analysis (HPLC: absolute calibration curve method). The results are shown in Table 5 below. The pH of the contents at the start of preservation was about 10 as measured using a pH test paper.

### Comparative Examples 5 and 6 (Preservation stability of high-purity piperonyl alcohol: method (2); anti-decomposition agent: none)

Preservation was performed by the same method as respectively in Examples 10 and 11 except that no anti-decomposition agent was added at a preservation temperature of 90°C (Comparative Example 5) or 45°C (Comparative Example 6), and similarly, 7 days, 14 days, 21 days, and 28 days after the start of preservation, a purity of piperonyl alcohol was measured by a high performance liquid chromatography analysis (HPLC: absolute calibration curve method). The results are shown in Table 5.

**[Table 5]**

| Preservative stability of high-purity piperonyl alcohol | | | | |
|---|---|---|---|---|
| | Preservation temperature (°C) | Preservation time (Days) | Anti-decomposition agent (Sodium carbonate: 5,000 ppm) | |
| | | | Added *1 | None *3 |
| | | | Purity (wt%)*2 | Purity (wt%) |
| Example 10 (Comparative Example 5) | 90°C | 0 (Start) | 95.0 | 95.0 |
| | | 7 | 95.3 | 57.4 |
| | | 14 | 96.5 | 47.0 |
| | | 21 | 95.5 | 41.4 |
| | | 28 | 97.8 | 39.2 |
| Example 11 (Comparative Example 6) | 45°C | 0 (Start) | 99.6 | 99.6 |
| | | 7 | 97.6 | 93.5 |
| | | 14 | 98.0 | 88.9 |
| | | 21 | 94.6 | 90.0 |
| | | 28 | 96.4 | 89.4 |

| | | | | |
|---|---|---|---|---|
| * 1: High-purity piperonyl alcohol composition. *2: Purity of piperonyl alcohol (% by mass: wt%), as measured by high performance liquid chromatography analysis (HPLC: absolute calibration curve method). *3: Experimental results of Comparative Example (anti-decomposition agent: none). | | | | |

### Examples 12 to 15 (Preservative stability of high-purity piperonyl alcohol: method (2); anti-decomposition agent: sodium carbonate)

Using 10 g of high-purity piperonyl alcohol (piperonyl alcohol purity: 96.6 wt%; HPLC: absolute calibration curve method; formula (3) purity: 0 wt%) obtained by the same method as in Example 2, preservation was started at a preservation temperature of 90°C by the same method as in Example 10 except that the amount of the anti-decomposition agent (sodium carbonate) used was changed to 200 ppm (Example 12), 500 ppm (Example 13), 750 ppm (Example 14), or 1,000 ppm (Example 15). The pH of the contents at the start of preservation was about 10 as measured using a pH test paper.
After a lapse of 30 days, with respect to each sample, a purity of piperonyl alcohol (in terms of % by mass) was measured by a high performance liquid chromatography analysis (HPLC: absolute calibration curve method). The results are shown in Table 6 below.

**[Table 6]**

| Preservative stability of high-purity piperonyl alcohol (90°C) | | | |
|---|---|---|---|
| | Stabilizer (ppm) (Sodium carbonate) | Piperonyl alcohol purity (wt%)*1 | Bis(3,4-methylenedioxybenzyl) ether (formula (3d)) content (wt%)*2 |
| Example 12 | 200 | 91.6 | 1.38 |
| Example 13 | 500 | 92.7 | 0.59 |
| Example 14 | 750 | 94.8 | 0.51 |
| Example 15 | 1000 | 95.7 | 0.44 |

| | | | |
|---|---|---|---|
| *1: Purity of piperonyl alcohol (% by mass: wt%), as measured by high performance liquid chromatography analysis (HPLC: absolute calibration curve method). *2: Bis(3,4-methylenedioxybenzyl) ether (formula (3d)) content (% by mass: wt%), as measured by high performance liquid chromatography analysis (HPLC: absolute calibration curve method). | | | |

As seen from Tables 5 and 6 above, for example, with respect to the preservation under conditions at 90°C, when no anti-decomposition agent is added, half or more of piperonyl alcohol decomposes in 14 days, but, as shown in Example 11, when a anti-decomposition agent in an amount of 200 ppm or more is added, piperonyl alcohol can be stabilized, making it possible to store piperonyl alcohol for a long term.

### INDUSTRIAL APPLICABILITY

The present invention relates to a method for producing an aromatic methyl alcohol having high purity and a high-purity aromatic methyl alcohol composition having excellent preservative stability. The aromatic methyl alcohol obtained by the present invention is known to be used as various chemical products, such as medical or agricultural chemicals, and organic materials, or a raw material or intermediate thereof. Particularly, the piperonyl alcohol of the present invention is useful as a raw material for synthesis of, e.g., perfumery and cosmetics and insecticides.

## Claims

1. A method for producing a high-purity aromatic methyl alcohol, comprising a step of subjecting a crude product containing an aromatic methyl alcohol represented by the following general formula (1): 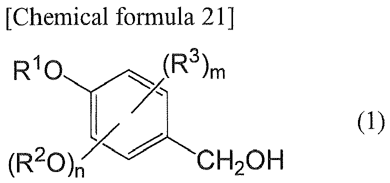 wherein
each of R¹ and R² represents a hydrogen atom, an alkyl group having 1 to 12 carbon atoms, a phenyl group, a benzyl group, an allyl group, or a propargyl group, each of which optionally has a substituent,
n is the number of substituent(s) OR² and represents an integer of from 0 to 3, and when n is 2 or more, R² may be the same or different, and when the substituents (OR¹ and OR²) on the aromatic ring are present on the adjacent carbons constituting the aromatic ring, R¹ and R² may be bonded together to form a cyclic structure,
R³ represents a halogen atom (a fluorine atom, a chlorine atom, a bromine atom, or an iodine atom), a nitro group, a cyano group, a methyl group, an ethyl group, a trifluoromethyl group, or a phenyl group, wherein when R³ and substituent OR¹ or OR² on the aromatic ring are present on the adjacent carbons constituting the aromatic ring, R³ and R¹ or R² may be bonded together to form a cyclic structure, and
m is the number of substituent(s) R³ and represents an integer of from 0 to 3, and when m is 2 or more, R³ may be the same
or different, with the proviso that n + m is an integer of from 0 to 4 to distillation in the presence of an anti-decomposition agent to obtain a high-purity aromatic methyl alcohol represented by the general formula (1).

2. The method according to claim 1, wherein the crude product containing an aromatic methyl alcohol is obtained by subjecting an aromatic methyl halide represented by the following general formula (2): 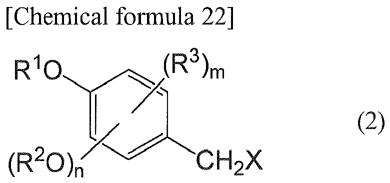 wherein R¹ to R³, n, and m are as defined for the general formula (1) in claim 1, and X represents a chlorine atom, a bromine atom, or an iodine atom
to hydrolysis reaction.

3. The method according to claim 2, wherein the aromatic methyl halide is selected from compounds represented by the following general formulae (2a) to (2g): 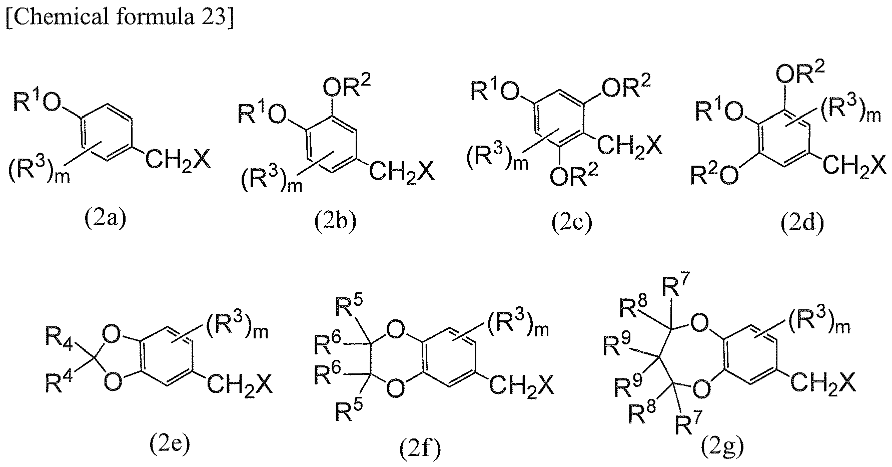 wherein R¹ to R³, X, and m are as defined for the formula (2) in claim 2, and R¹ to R³ may be the same or different, and
in the formulae (2e) to (2g), each of R⁴ to R⁹ represents a hydrogen atom, a fluorine atom, or a methyl group, and R⁴ to R⁹ may be the same or different.

4. The method according to claim 2 or 3, wherein the aromatic methyl halide is 4-methoxybenzyl chloride, 3,4-dimethoxybenzyl chloride, 3,4,5-trimethoxybenzyl chloride, 3,4-ethylenedioxybenzyl chloride, or 3,4-methylenedioxybenzyl chloride.

5. The method according to any one of claims 1 to 4, wherein the anti-decomposition agent is at least one member selected from the group consisting of an alkali metal carbonate compound, an alkaline earth metal carbonate compound, an alkali metal hydrogencarbonate compound, an alkali metal hydroxide, an alkaline earth metal hydroxide, an alkali metal phosphate compound, an alkaline earth metal phosphate compound, and an anion-exchange resin.

6. The method according to any one of claims 1 to 5, wherein the distillation is performed under conditions such that the liquid temperature in a distillation vessel is 70 to 240 °C

7. The method according to any one of claims 1 to 6, wherein the distillation is performed under conditions such that the pH of the contents of a distillation vessel is 8 to 14.

8. A high-purity aromatic methyl alcohol being represented by the following general formula (1): 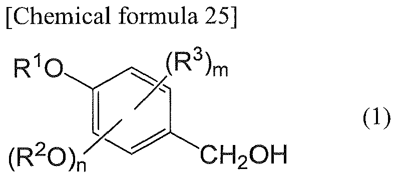 wherein R¹ to R³, n, and m are as defined for the general formula (1) in claim 1,
in which the content of a bis(arylmethyl) ether represented by the following general formula (3): 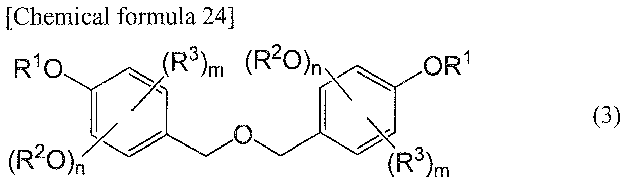 wherein R¹ to R³, n, and m are as defined for the general formula (1)
in claim 1
in the high-purity aromatic methyl alcohol is 10% or less, as determined using the following equation 1:
[Equation 4] $Content \left(%\right) of the bis \left(arylmethyl\right) ether represented by general formula \left(3\right) in the obtained high - purity aromatic methyl alcohol represented by general formula \left(1\right) = \frac{B}{\left(A+B\right)} \times 100 \left(%\right)$

9. The high-purity aromatic methyl alcohol according to claim 8, which is produced by the method according to any one of claims 1 to 7.

10. A high-purity aromatic methyl alcohol composition comprising a high-purity aromatic methyl alcohol represented by the following general formula (1): 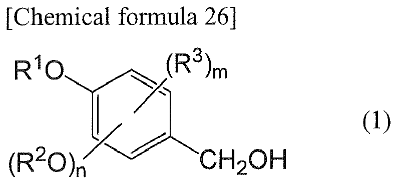 wherein R¹ to R³, n, and m are as defined for the general formula (1)
in claim 1,
and at least one anti-decomposition agent selected from the group consisting of an alkali metal carbonate compound, an alkaline earth metal carbonate compound, an alkali metal hydrogencarbonate compound, an alkali metal hydroxide, an alkaline earth metal hydroxide, an alkali metal phosphate compound, an alkaline earth metal phosphate compound, and an anion-exchange resin.

11. The high-purity aromatic methyl alcohol composition according to claim 10, wherein the high-purity aromatic methyl alcohol is the high-purity aromatic methyl alcohol according to claim 9.

12. The high-purity aromatic methyl alcohol composition according to claim 10 or 11, wherein the amount of the anti-decomposition agent used is 200 to 50,000 ppm, based on the mass of the pure aromatic methyl alcohol contained in the high-purity aromatic methyl alcohol.

13. A vessel having preserved therein the high-purity aromatic methyl alcohol composition according to any one of claims 10 to 12.

14. A method for storing a high-purity aromatic methyl alcohol, comprising a step of adding, as a preserving agent, at least one anti-decomposition agent selected from the group consisting of an alkali metal carbonate compound, an alkaline earth metal carbonate compound, an alkali metal hydrogencarbonate compound, an alkali metal hydroxide, an alkaline earth metal hydroxide, an alkali metal phosphate compound, an alkaline earth metal phosphate compound, and an anion-exchange resin, to a high-purity aromatic methyl alcohol represented by the following general formula (1): 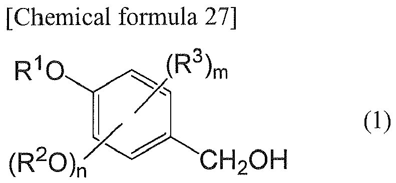 wherein R¹ to R³, n, and m are as defined for the general formula (1)
in claim 1,
to store the high-purity aromatic methyl alcohol.

15. A method for distilling an aromatic methyl alcohol, comprising a step of subjecting a crude product containing an aromatic methyl alcohol represented by the following general formula (1): 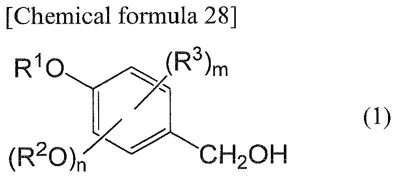 wherein R¹ to R³, n, and m are as defined for the general formula (1) in claim 1
to distillation for purification of the aromatic methyl alcohol in the presence of at least one anti-decomposition agent selected from the group consisting of an alkali metal carbonate compound, an alkaline earth metal carbonate compound, an alkali metal hydrogencarbonate compound, an alkali metal hydroxide, an alkaline earth metal hydroxide, an alkali metal phosphate compound, an alkaline earth metal phosphate compound, and an anion-exchange resin.

16. The method for distilling an aromatic methyl alcohol according to claim 15, wherein the distillation is performed under conditions such that the pH of the contents of a distillation vessel is 8 to 14.

17. The method for distilling an aromatic methyl alcohol according to claim 15 or 16, wherein the crude product containing an aromatic methyl alcohol is a crude product containing an aromatic methyl alcohol obtained by subjecting to hydrolysis reaction an aromatic methyl halide represented by the following general formula (2): 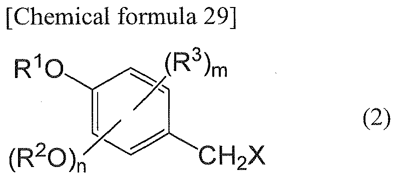 wherein R¹ to R³, n, m, and X are as defined for the general formula (2) in claim 2.

18. A method for stabilizing a high-purity aromatic methyl alcohol, comprising a step of adding, as a stabilizing agent, at least one anti-decomposition agent selected from the group consisting of an alkali metal carbonate compound, an alkaline earth metal carbonate compound, an alkali metal hydrogencarbonate compound, an alkali metal hydroxide, an alkaline earth metal hydroxide, an alkali metal phosphate compound, an alkaline earth metal phosphate compound, and an anion-exchange resin to a high-purity aromatic methyl alcohol represented by the following general formula (1): 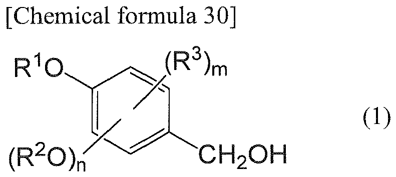 wherein R¹ to R³, n, and m are as defined for the general formula (1) in claim 1.

19. The method for stabilizing a high-purity aromatic methyl alcohol according to claim 18, which comprises a step of adding the stabilizing agent to the high-purity aromatic methyl alcohol so that the pH becomes 8 to 14.

20. A preserving agent for an aromatic methyl alcohol represented by the following general formula (1): 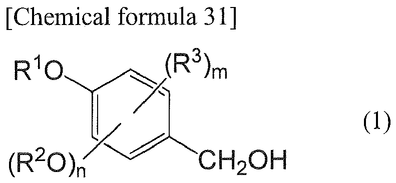 wherein R¹ to R³, n, and m are as defined for the general formula (1) in claim 1,
comprising at least one member selected from the group consisting of an alkali metal carbonate compound, an alkaline earth metal carbonate compound, an alkali metal hydrogencarbonate compound, an alkali metal hydroxide, an alkaline earth metal hydroxide, an alkali metal phosphate compound, an alkaline earth metal phosphate compound, and an anion-exchange resin.

21. A stabilizing agent for an aromatic methyl alcohol represented by the following general formula (1): 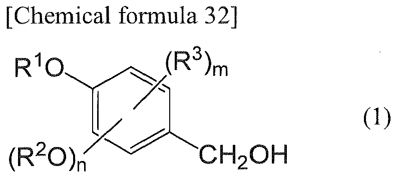 wherein R¹ to R³, n, and m are as defined for the general formula (1)
in claim 1,
the stabilizing agent comprising at least one member selected from the group consisting of an alkali metal carbonate compound, an alkaline earth metal carbonate compound, an alkali metal hydrogencarbonate compound, an alkali metal hydroxide, an alkaline earth metal hydroxide, an alkali metal phosphate compound, an alkaline earth metal phosphate compound, and an anion-exchange resin.
